# EUROPÄISCHE PATENTSCHRIFT

(11) **EP 2 842 933 B1**
(45) Veröffentlichungstag und Bekanntmachung des Hinweises auf die Patenterteilung: **29.07.2015**
(21) Anmeldenummer: 13182788.3
(22) Anmeldetag: 03.09.2013
(51) Int. Cl.: C07C 67/03, C07D 311/80, C07C 69/94, A61K 31/353, A61K 31/25, A61P 37/04, A61P 29/00, A61P 25/24, A61P 27/06, A61P 25/06, A61P 1/14, A61P 1/08

(54) **Mischungen cannabinoider Verbindungen, deren Herstellung und Verwendung**
Mixtures of cannabinoid compounds, their preparation and use
Mélanges de composés cannabinoïdes, leur préparation et leur utilisation

(43) Veröffentlichungstag der Anmeldung: 04.03.2015
(73) Patentinhaber: Symrise AG, 37603 Holzminden (DE)
(72) Erfinder: Koch, Oskar, 37079 Göttingen (DE); Götz, Marcus, Rudolf, 34399 Oberweser (DE); Looft, Jan, Dr., 53343 Wachtenberg-Niederbachem (DE); Vössing, Tobias, 37688 Beverungen (DE)
(74) Vertreter: Eisenführ Speiser

(56) Entgegenhaltungen:
- DE-A1-102009 019 322
- A. F. ARTAMONOV ET AL: "Synthesis of [alpha]-monoglycerides of aromatic acids", CHEMISTRY OF NATURAL COMPOUNDS, Bd. 35, Nr. 4, 1. Juli 1999 (1999-07-01), Seiten 404-408, XP055090710, ISSN: 0009-3130, DOI: 10.1007/BF02282504
- BELA SZABO: "Pharmacology of Cannabinoid Receptors", INTERNET CITATION, 1. Januar 2008 (2008-01-01), Seiten 1-13, XP002638928, Gefunden im Internet: URL:http://www.slideshare.net/qnbs7/pharma cology-of-cannabinoid-receptors [gefunden am 2011-06-06]

## Beschreibung

Die vorliegende Erfindung betrifft spezifische Mischungen umfassend eine oder mehrere (cannabinoide) Verbindungen der Formel (A) und/oder ein oder mehrere von deren Salzen 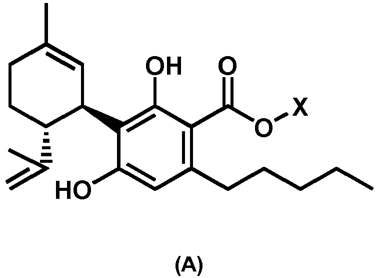 sowie Verfahren zu deren Herstellung. Hinsichtlich der Bedeutung des Substituenten X siehe unten.

Die Erfindung betrifft auch eine Verbindung der obigen Formel (A), ein Salz der Formel (A) und eine Mischung umfassend eine oder mehrere (cannabinoide) Verbindungen der Formel (A) und/oder ein oder mehrere von deren Salzen, jeweils zur Anwendung als Arzneimittel bzw. zur Anwendung in einem Verfahren zur therapeutischen Behandlung des menschlichen oder tierischen Körpers.

Außerdem betrifft die vorliegende Erfindung eine Verbindung der Formel (A) bzw. ein Salz der Formel (A) bzw. eine Mischung umfassend eine oder mehrere cannabinoide Verbindungen der Formel (A) und/oder ein oder mehrere von deren Salzen zur spezifischen Anwendung in einem Verfahren zur therapeutischen Behandlung des menschlichen oder tierischen Körpers zum Erreichen einer Wirkung ausgewählt aus der Gruppe bestehend aus appetitanregende Wirkung, antiemetische Wirkung zur Hemmung von Übelkeit und Erbrechen, Reduktion muskulärer Krämpfe und Spastiken, Linderung von Schmerzsymptomen, Linderung von Migränesymptomen, Senkung des Augeninnendrucks beim Glaukom, Stimmungsaufhellung, Immunstimulation und/oder antiepileptische Wirkung.

Die vorliegende Erfindung betrifft zudem eine pharmazeutische Formulierung, umfassend eine oder mehrere Verbindungen der Formel (A) oder umfassend ein oder mehrere von deren Salzen oder umfassend eine Mischung umfassend eine oder mehrere (cannabinoide) Verbindungen der Formel (A) und/oder ein oder mehrere von deren Salzen, ausgewählt aus der Gruppe bestehend aus feste galenische Formen, Dragees, Kapseln, Granulate, Pulver, Suppositorien, Lutschbonbons, Kaugummis, halbfeste Formen, Inhalate, Injektabilia, Implantate und wirkstoffhaltige Pflaster.

Die vorliegende Erfindung betrifft auch ein Verfahren zur Herstellung von Delta-9-Tetrahydrocannabinol (Delta-9-THC).

Weitere Aspekte der vorliegenden Erfindung ergeben sich aus der nachfolgenden Beschreibung sowie den beigefügten Patentansprüchen.

Seit der Entdeckung des endogenen Cannabinoid-Systems mit seiner funktionellen Bedeutung für die Regulation und Modulation des Immun- sowie Nervensystems besteht ein ständiger Bedarf an natürlichen und künstlichen Cannabinoiden zu dessen selektiver pharmazeutischen Steuerung. Insbesondere besteht Bedarf, die Cannabinoid-Rezeptoren CB1, welche vor allem in Nervenzellen, in höchster Dichte in den Basalganglien, im Hippocampus und im Cerebellum, zu finden sind, und die Cannabinoid-Rezeptoren CB2, welche vorwiegend auf Zellen des Immunsystems und auf am Knochenaufbau und -abbau beteiligten Zellen zu finden sind, aufgrund ihrer unterschiedlichen medizinischen Funktionen gezielt separat zu stimulieren.

Die Cannabinoid-Rezeptoren CB1 und CB2 gelten als akzeptierte Wirkorte für Moleküle mit cannabinoider Struktur. Obwohl noch weitere Rezeptoren als potentieller CB3 Rezeptor diskutiert werden, wird davon ausgegangen, dass die Hauptwirkungen durch CB1 und CB2 vermittelt werden. Delta-9-THC, endogene Cannabinoide und eine Vielzahl synthetischer Cannabinoide kuppeln an die genannten Rezeptoren und üben über sie Effekte auf die Zellen aus (Pertwee, R. G. et al. Pharmacol. Rev. 2010, 62, 588-631).

CB1 und CB2 sind Mitglieder der Superfamilie der G-Protein-gekoppelten Rezeptoren (GPCRs, G Protein Coupled Receptors). Genauer gesagt hemmen die Rezeptoren über das heteroemere G-Protein die Andenylatcyclase und aktivieren die mitogen aktivierte Protein-Kinase (Howlett, A. C. et al. Pharmacol. Rev. 2002, 54, 161-202; Howlett, A. C. Handb. Exp. Pharmacol. 2005, 168, 53-79). Für den CB1-Rezeptor ist weiterhin beschrieben, dass er über Ionen-Kanäle des A-Typs Kalium Ströme und über N sowie P/Q-Typ Kanäle Calcium Ströme modulieren kann. Weiterhin können CB1-Rezeptoren über Gₛ-Proteine Signale an die exprimierenden Zellen übertragen (Glass, M.,Felder, C. C. J. Neurosci. 1997; 17, 5327-5333; Maneuf, Y. P., Brotchie, J. M. J. Pharmacol. 1997; 120, 1397-1398; Calandra, B. et al. Eur. J. Pharmacol. 1999; 374, 445-455; Jarrahian, A. et al. J. Pharmacol. Exp. Ther. 2004, 308, 880-886).

Die Fähigkeit von CB1 und CB2 über G_{i/o} und im weiteren Downstream über die Hemmung der Adenylatcyclase Signale zu vermitteln, wird im sogenannten [³⁵S]GTP gammaS binding assay und dem cAMP assay (Howlett, A. C. et al. Pharmacol. Rev. 2002, 54, 161-202; Pertwee, R. G. Handb. Exp. Pharmacol. 2005a, 168, 1-51) verwendet, um die Bindung und die Signalübertragung von Cannabinoiden zu untersuchen.

CB1-Rezeptoren verfügen sowohl über eine orthosterische als auch über eine oder mehrere allosterische Bindungsstellen, die als potentielle Wirkorte für Liganden in Frage kommen (Price, M. R. et al. Mol. Pharmacol. 2005a, 68, 1484-1495; Adam, L. et al. 17th Annual Symposium of the Cannabinoids, 2007, S. 86; Horswill, J. G. et al. J. Pharmacol. 2007, 152, 805-814; Navarro, H. A. et al. J. Pharmacol. 2009, 156, 1178-1184). CB1-Rezeptoren werden hauptsächlich an den terminalen Enden von zentralen und peripheren Nervenzellen gefunden, wo sie üblicherweise eine Hemmung von exzitatorischen und inhibitorischen Neurotransmittern vermitteln (Howlett, A. C. et al. Pharmacol. Rev. 2002, 54, 161-202; Pertwee, R. G., Ross, R. A. Prostaglandins Leukot Essent Fatty Acids, 2002, 66, 101-121; Szabo, B., Schlicker, E. Handb. Exp. Pharmacol. 2005, 168, 327-365). Die Verteilung dieser Rezeptoren im zentralen Nervensystem ist dergestalt, dass ihre Aktivierung verschiedene kognitive Prozesse beeinflussen können (z.B. die Aufmerksamkeit und das Gedächtnis, verschiedene motorische Funktionen und die Schmerzwahrnehmung).

CB2-Rezeptoren sind vornehmlich wie bereits erwähnt in Immunzellen lokalisiert. Werden sie aktiviert, modulieren Sie die Zellmigration und die Ausschüttung von Cytokinen innerhalb und außerhalb des Gehirns (Howlett, A. C. et al. Pharmacol. Rev. 2002, 54, 161-202; Cabral, G. A., Staab, A. Handb. Exp. Pharmacol. 2005, 168, 385-423; Pertwee, R. G. Handb. Exp. Pharmacol. 2005a, 168, 1-51).

Es gibt außerdem Nachweise dafür, dass erstens CB1-Rezeptoren von nicht-neuronalen Zellen (einschließlich Immunzellen) (Howlett, A. C. et al. Pharmacol. Rev. 2002, 54, 161-202) und dass zweitens CB2-Rezeptoren von manchen Zellen innerhalb und außerhalb des Gehirns exprimiert werden (Skaper, S. D. et al. Proc. Natl. Acad. Sci. USA 1996, 93, 3984-3989; Ross, R. A. et al. Neuropharmacology 2001a, 40, 221-232; Van Sickle, M. D. et al. Science 2005, 310, 329-332; Wotherspoon, G. et al. Neuroscience 2005, 135, 235-245; Beltramo, M. et al. Eur. J. Neurosci. 2006, 23, 1530-1538; Gong, J. P. et al. Brain Res. 2006, 1071, 10-23; Baek, J. H. et al. Acta Otolaryngol 2008, 128, 961-967).

Bekannte Verbindungen, die nachgewiesenermaßen eine Affinität zu den oben genannten Rezeptoren CB1 und CB2 aufweisen, sind das u.a. aus den Vertretern des weiblichen Hanfs *Cannabis sativa* und *Cannabis indica* stammende Cannabidiol (CBD) sowie bestimmte chemische Derivate wie Delta-8- und Delta-9-Tetrahydrocannabinol (Delta-9-THC) oder dessen Oxidationsprodukt Cannabinol (CBN).

Cannabis gehört zur Familie der Cannabidaceae. Die botanische und chemotaxonomische Einteilung der Gattung Cannabis geschieht anhand zweier unterschiedlicher Vorgehensweisen. Schultes et al. unterscheidet drei Arten Cannabis sativa Linnaeus, Cannabis indica LAM. und Cannabis ruderalis (Schultes, R. E. et al. Harvard University Botanical Museum Leaflets 1974, 23, 337-367). Andere benennen nur die eine Sammelart Cannabis sativa L. aus den Unterarten Cannabis sativa ssp. sativa und ssp. indica.

Nach der fachjuristischen Sichtweise wird in einen Drogen- und einen Fasertyp unterschieden, wobei die Differenzierung aufgrund des mengenmäßigen Verhältnisses der Hauptcannabinoide CBD und Delta-9-THC geschieht.

Aus dem Stand der Technik sind verschiedene cannabinoide Verbindungen und Verfahren zur deren Herstellung bekannt. So beschreibt WO 2006/136273 ein Verfahren zur Herstellung von Dronabinol ((im WO-Dokument bezeichnet als: (6aR-trans)-6a,7,8,10a-tetrahydro-6,6,9-trimethyl-3-pentyl-6H-dibenzo[b,d]pyran-1-ol, Δ⁹-Tetrahydrocannabinol (Δ⁹-THC)), heute auch gemäß IUPAC bezeichnet als (6a*R*,10a*R*)-6,6,9-Trimethyl-3-pentyl-6a,7,8,10a-tetrahydro-6*H*-benzo[c]chromen-1-ol oder bezeichnet als Delta-9-Tetrahydrocannabinol, Delta-9-THC oder Δ-9-THC) aus Cannabidiol (CBD) durch Cyclisierung von Cannabidiol (CBD) (2-[1R-3-Methyl-6-(1-methylethenyl)-2-cyclohexen-1-yl]-5-pentyl-1,3-benzoldiol) zu Delta-9-THC. Das beschriebene Verfahren ist dadurch gekennzeichnet, dass Cannabidiol (CBD) in einem organischen Lösungsmittel vorgelegt und in Gegenwart eines Molekularsiebs unter Erhitzen zu Delta-9-THC cyclisiert wird. Es wird in der WO 2006/136273 festgestellt, dass das eingesetzte Molekularsieb, neben den bisher beschriebenen trocknenden Eigenschaften, auch starke katalytische Eigenschaften besitzt, die bei der beschriebenen Umsetzung im Vordergrund stehen. Cyclisierungen, die nur in Gegenwart eines Lewis-Säure Katalysators ausgeführt werden, sind in der Regel deutlich langsamer und liefern schlechtere Ausbeuten an Delta-9-THC, als Cyclisierungen, die in Gegenwart eines Molekularsiebes durchgeführt werden.

In der Literatur sind weitere Synthesevarianten beschrieben wie z.B. von Crombie et al. Chem. Research 1977, 114, 1301-1345. Neuere Syntheseverfahren werden u.a. in EP 2314580 offenbart. Das dort beschriebene Verfahren zur Herstellung von Cannbinoiden soll anwendbar sein für alle Stereoisomeren und Homologen von Cannabinoiden und besteht aus zwei bzw. drei chemischen Syntheseschritten. Dabei werden in einem ersten Schritt Alkylresorcylsäureester (6-Alkyl-2,4-dihydroxybenzoesäureester) mit ungesättigten Kohlenwasserstoffen, Alkoholen, Ketonen (bzw. deren Derivaten wie Enolestern, Enolethern und Ketalen) zu den entsprechenden in 3-Stellung substituierten 6-Alkyl-2,4-dihydroxybenzossäureestern kondensiert. In einem zweiten Schritt werden die im ersten Schritt erzeugten Zwischenprodukte mit Esterfunktion einer decarboxylierenden Verseifung unterzogen, wodurch die entsprechenden esterfreien Cannabinoide entstehen. Sofern nötig wird in einem dritten Schritt eine sauer katalysierte Umlagerung vorgenommen. Diese Isomerisierung kann z. B. der Ringschluss des Pyranrings bei CBD zu Dronabinol sein, aber auch die Umlagerung einer Doppelbindung wie, z. B. die Umwandlung von Delta-9- in Delta-8-THC oder eine sauer katalysierte Epimerisierung wie die Umlagerung von cis-9-Ketocannabinoiden in die entsprechenden trans-Verbindungen.

US 5,342,971 beschreibt ein Verfahren zur Herstellung von Dronabinol und verwandten Dibenzo[b,d]pyranen. Diese werden gemäß des Abstracts durch Erhitzen eines Dihydroxybenzoesäurederivates in Gegenwart eines Lewis-Säure Katalysators und eines inerten unpolaren Lösungsmittels, in welchem zwar die Dihydroxybenzoesäure löslich, der Lewis-Säure Katalysator jedoch unlöslich oder sehr geringfügig löslich ist, hergestellt. Eine typische Ausgestaltung beinhaltet die Herstellung von für die Synthese von Dronabinol und verwandten Dibenzo[b,d]pyranen nützlichen Intermediaten.

Delta-9-THC ist z.B. als wirksame Substanz in dem Arzneimittel *Marinol^{®}* in den USA seit 1985 zugelassen gegen Anorexie, die bei Patienten unter einer AIDS Therapie vorkommt, sowie gegen Nausea und Emesis, die im Zusammenhang mit der Chemotherapie bei Krebspatienten vorkommt (Tumorkachexie).

In Deutschland steht Delta-9-THC in Anlage III des Betäubungsmittelgesetzes (BtMG) und kann ohne Indikationseinschränkungen auf Betäubungsmittelrezept verschrieben werden. Da jedoch kein Fertigarzneimittel im Handel verfügbar ist, kann entweder ausnahmsweise eine Verschreibung von Dronabinol in Form von *Marinol^{®}* und damit der Import aus dem Ausland geschehen, oder eine Rezepturherstellung über eine Apotheke nach anerkannten pharmazeutischen Regeln vorgenommen werden.

Zudem ist als Fertigarzneimittel in Deutschland seit Mai 2011 ein Extrakt aus Cannabis sativa unter dem Namen *Sativex*® zugelassen. Die Zulassung bezieht sich auf die Zusatzbehandlung zur Symptomverbesserung bei Patienten mit mittelschwerer bis schwerer Spastik aufgrund von Multipler Sklerose, die nicht angemessen auf eine andere anti-spastische Arzneimitteltherapie angesprochen haben. Das Arzneimittel enthält eine Wirkstoffkombination aus Delta-9-THC und Cannabidiol und wird auf einem Betäubungsmittelrezept (BtM-Rezept) verordnet. Es wird als Spray in der Mundhöhle angewendet.

Die Verordnung Nr. 1164/89 der europäischen Kommission bezeichnet Hanf mit einem (Delat-9-THC) Gehalt von bis zu 0,3 % bezogen auf die Trockenmasse als Nutzhanf wohingegen sogenannter Drogenhanf einen Gehalt von 5% - 15% haben kann.

Neben der extraktiven Isolierung aus Hanf ist auch eine Teilsynthese aus Cannabidiol möglich. Diese Vorstufe lässt sich aus Faserhanf isolieren und dann säurekatalytisch zum Delta-9-Tetrahydrocannabinol cyclisieren wie dies z.B. in WO 2006/136273 beschrieben wird.

DE 10 2009 019 322 offenbart Cannabidiolsäurealkylester, die als Zwischenprodukte in der Synthese von Cannabinoiden verwendet werden.Diese Verbindungen besitzen keine hydroxylierte Alkoholseitenketten.

A. F. ARTAMONOV ET AL: "Synthesis of [alpha]-monoglycerides of aromatic acids", CHEMISTRY OF NATURAL COMPOUNDS,Bd. 35, Nr. 4, 1. Juli 1999 (1999-07-01), Seiten 404-408, offenbart Cannabidiol als Antagonisten der CB1 und CB2 Rezeptoren.

Eine Aufgabe der vorliegenden Erfindung war es, cannabinoid wirksame Stoffe oder Stoffgemische (und Verfahren zu deren Herstellung) anzugeben, die eine starke CB1-bzw. CB2-Affinität aufweisen, wobei vorzugsweise eine der beiden genannten Rezeptoraffinitäten gegenüber der anderen überwiegt. Das anzugebende Verfahren sollte vorzugsweise eine gute Raum-Zeit-Ausbeute in Verbindung mit ökologischen Vorteilen (bevorzugt Verwendung nicht-chlorhaltiger Lösungsmittel) besitzen.

Die anzugebenden Stoffe oder Stoffgemische sollten dabei vorzugsweise als Arzneimittel oder in einem Verfahren zur therapeutischen Behandlung des menschlichen oder tierischen Körpers zum Erreichen einer Wirkung ausgewählt aus der Gruppe bestehend aus appetitanregende Wirkung, antiemetische Wirkung zur Hemmung von Übelkeit und Erbrechen, Reduktion muskulärer Krämpfe und Spastiken, Linderung von Schmerzsymptomen, Linderung von Migränesymptomen, Senkung des Augeninnendrucks beim Glaukom, Stimmungsaufhellung, Immunstimulation und/oder antiepileptischen Wirkung angewendet werden können.

Die vorliegende Erfindung basiert u.a. auf der überraschenden Erkenntnis, dass Verbindungen der Formel (A) sowie deren Salze, wobei der Substituent X in der Formel (A) ein aliphatischer Rest mit einer, zwei, drei oder mehr als drei Hydroxygruppen ist, wobei die Gesamtzahl der C-Atome in dem aliphatischen Rest X nicht größer ist als 15, vorzugsweise nicht größer ist als 12, und
wobei der aliphatische Rest
- gesättigt oder ungesättigt ist
   und
- verzweigt oder unverzweigt ist,
   wobei die Verbindung der Formel (A) ausgewählt ist aus der Gruppe bestehend aus: 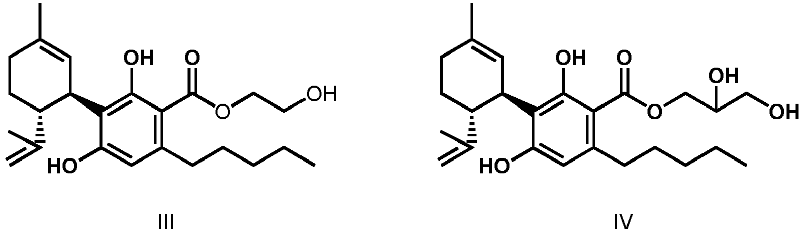 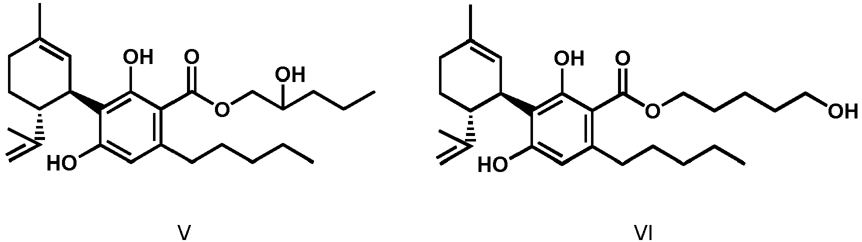 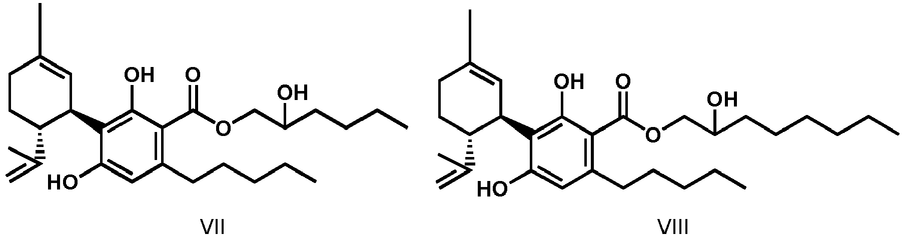 eine günstige und einzigartige Bindungsaffinität gegenüber den Cannabinoid-Rezeptoren CB1 und CB2 aufweisen, wodurch sie sich zur Anwendung als Arzneimittel oder zur Anwendung in einem Verfahren zur therapeutischen Behandlung des menschlichen oder tierischen Körpers eignen.

Die Anwendung einer oder mehrerer Verbindungen der Formel (A) (wie vorstehend und nachfolgend, insbesondere in den Ansprüchen definiert) oder eines oder mehrerer von deren Salzen oder einer entsprechenden Mischung (wie vorstehend und nachfolgend, insbesondere in den Ansprüchen definiert) als Arzneimittel bzw. in einem Verfahren zur therapeutischen Behandlung des menschlichen oder tierischen Körpers zielt dabei insbesondere auf das Erreichen einer Wirkung ausgewählt aus der Gruppe bestehend aus
- appetitanregende Wirkung,
- antiemetische Wirkung zur Hemmung von Übelkeit und Erbrechen,
- Reduktion muskulärer Krämpfe und Spastiken,
- Linderung von Schmerzsymptomen
- Linderung von Migränesymptomen,
- Senkung des Augeninnendrucks beim Glaukom,
- Stimmungsaufhellung
- Immunstimulation
   und/oder
- antiepileptische Wirkung.

In eigenen Untersuchungen wurden insbesondere die folgenden Stoffe III-V der allgemeinen Formel (A) auf ihre Wirkung an Cannabinoid-Rezeptoren untersucht.

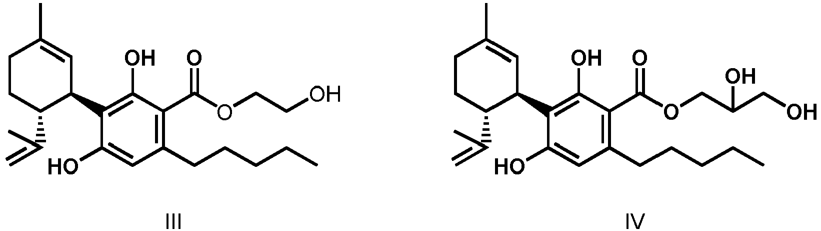

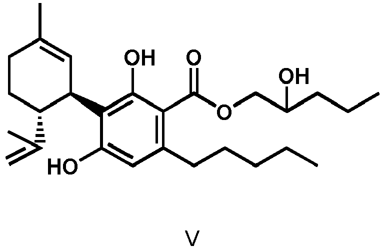

Die Stoffe III-V wurden in Kompetitions-Studien auf Ihre Bindungsaffinität und ihr resultierendes Bindungsprofil zu CB1- und CB2-Rezeptoren untersucht. Für Details sei insoweit auf die Beispiele weiter unten verwiesen. Die Studien haben insbesondere ergeben, dass die cannabinoiden Substanzen III-V in nM-Konzentrationen und damit in physiologischen Dosen an Cannabinoid-Rezeptoren binden. Sie sind schwache Liganden an CB1-Rezeptoren und binden bevorzugt an CB2-Rezeptoren. Ihre Selektivität für CB2-Rezeptoren prädestiniert sie für den Einsatz als CB2-Rezeptormodulatoren.

Verbindungen der Formel (A) (wie vorstehend und nachfolgend, insbesondere in den Ansprüchen definiert), lösen somit aufgrund ihrer spezifischen CB1- bzw. CB2-Rezeptoraffinität die oben beschriebene Aufgabe, vgl. hierzu erneut die Beispiele weiter unten.

Die Erfindung betrifft somit eine Verbindung der Formel (A) (wie vorstehend und nachfolgend, insbesondere in den Ansprüchen definiert) oder ein Salz einer Verbindung der Formel (A) (wie vorstehend und nachfolgend, insbesondere in den Ansprüchen definiert) oder einer Mischung (wie vorstehend und nachfolgend, insbesondere in den Ansprüchen definiert)
(i) zur Anwendung als Arzneimittel
   oder
(ii) zur Anwendung in einem Verfahren zur therapeutischen Behandlung des menschlichen oder tierischen Körpers.

Bevorzugt ist eine solche Verbindung der Formel (A) (wie vorstehend und nachfolgend, insbesondere in den Ansprüchen definiert) oder ein solches Salz einer Verbindung der Formel (A) (wie vorstehend und nachfolgend, insbesondere in den Ansprüchen definiert) oder einer solchen Mischung (wie vorstehend und nachfolgend, insbesondere in den Ansprüchen definiert) zur spezifischen Anwendung in einem Verfahren zur therapeutischen Behandlung des menschlichen oder tierischen Körpers, zum Erreichen einer Wirkung ausgewählt aus der Gruppe bestehend aus
- appetitanregende Wirkung,
- antiemetische Wirkung zur Hemmung von Übelkeit und Erbrechen,
- Reduktion muskulärer Krämpfe und Spastiken,
- Linderung von Schmerzsymptomen
- Linderung von Migränesymptomen,
- Senkung des Augeninnendrucks beim Glaukom,
- Stimmungsaufhellung
- Immunstimulation
   und/oder
- antiepileptische Wirkung.

Die Erfindung betrifft zudem eine pharmazeutische Formulierung, umfassend eine oder mehrere Verbindungen der Formel (A) (wie vorstehend und nachfolgend, insbesondere in den Ansprüchen definiert) oder umfassend ein oder mehrere von deren Salzen (wie vorstehend und nachfolgend, insbesondere in den Ansprüchen definiert) oder umfassend eine entsprechende Mischung (wie vorstehend und nachfolgend, insbesondere in den Ansprüchen definiert). Die erfindungsgemäße pharmazeutische Formulierung ist dabei vorzugsweise ausgewählt aus der Gruppe bestehend aus
- feste galenische Formen,
- Dragees,
- Kapseln,
- Granulate,
- Pulver,
- Suppositorien,
- Lutschbonbons,
- Kaugummis,
- halbfeste Formen,
- Inhalate,
- Injektabilia
- Implantate
   und
- wirkstoffhaltige Pflaster.

Alternativ liegt die pharmazeutische Formulierung in flüssiger Form vor.

Bevorzugte pharmazeutische Formulierungen sind:
Feste galenische Formen (wie z.B. Tabletten (mit Überzug und ohne, mit modifizierter Freisetzung und ohne), Dragees (mit Überzug und ohne, mit modifizierter Freisetzung und ohne), Kapseln (Weich- oder Hartgelatinekapseln mit modifizierter Freisetzung und ohne), Granulate (mit modifizierter Freisetzung und ohne), Pulver (mit modifizierter Freisetzung und ohne, z.B. Nasenpulver, Ohrenpuder), Suppositorien (mit Überzug und ohne, mit modifizierter Freisetzung und ohne), Lutschbonbons, Kaugummis, halbfeste Formen (wie z.B. hydrophobe Salben darunter z.B.: Kohlenwasserstoffgele, Lipogele, Silikongele, Oleogele sowie wasseraufnehmende Salben darunter z.B. Absorptionsbasen, hydrophile Salben, hydrophile Gele (Hydrogele) oder Pasten, auch Nasensalben), Inhalate (wie z.B. Druckgasdosierinhalatoren, Pulver-Inhalatoren, Inhalatoren mit Zerstäuber, Inhalationskonzentrate zur Inhalation), Injektabilia und Implantate (z.B. auf Basis flüssiger Formen oder fester Formen, die zur Zubereitung von oder selbst als injektionsfähigen Lösungen geeignet sind, oder feste Matrizen, die eine modifizierte Freisetzung möglich machen), wirkstoffhaltige Pflaster, Ohrentampons.

Flüssige Formen sind z.B. Lösungen, Suspensionen, Emulsionen, Sirupe (umgangssprachlich Hustensaft), Mundspülungen, Gurgellösungen, Halssprays oder Nasensprays, Nasentropfen, Nasenspüllösungen, Ohrentropfen, Ohrensprays und Ohrenspüllösungen. Pharmazeutische Formulierungen umfassend eine oder mehrere Verbindungen der Formel (A) (wie vorstehend und nachfolgend, insbesondere in den Ansprüchen definiert) und/oder ein oder mehrere von deren Salzen (wie vorstehend und nachfolgend, insbesondere in den Ansprüchen definiert) und/oder entsprechende Mischungen (wie vorstehend und nachfolgend, insbesondere in den Ansprüchen definiert) zur Anwendung als Arzneimittel oder zur Anwendung in einem Verfahren zur therapeutischen Behandlung des menschlichen oder tierischen Körpers enthalten vorzugsweise eine oder mehrere Bestandteile aus den folgenden Gruppen: Füllstoffe (z.B. Cellulose, Calciumcarbonat), Fließ- und Rieselmittel (z.B. Talkum, Magnesiumstearat), Überzüge (z.B. Polyvinylacetatphtalat, Hydroxypropylmethylcellulosephtalat), Sprengmittel (z.B. Stärke, quervernetztes Polyvinylpyrrolidon), Weichmacher (z.B. Triethylcitrat, Dibutylphtalat) Stoffe zur Granulierung (Lactose, Gelatine), Retardierung (z.B. Poly(meth)acrylsäure-methyl/ethyl/2-trimethyl-aminoethylester-Copolymerisate in Dispersion, Vinylacetat/ Crotonsäure-Copolymerisate), Kompaktierung (z.B. Mikrokristalline Cellulose, Lactose), Lösungs-, Suspendier- oder Dispergiermittel (z.B. Wasser, Ethanol), Emulgatoren (z.B. Cetylalkohol, Lecithin), Stoffe zur Veränderung der rheologischen Eigenschaften (Siliciumdioxid, Natriumalginat), Stoffe zur mikrobiellen Stabilisierung (z.B. Benzalkoniumchlorid, Kaliumsorbat), Konservierungsmittel und Antioxidantien (z.B. DLalpha-Tocopherol, Ascorbinsäure), Stoffe zur Veränderung des pH-Wertes (Milchsäure, Citronensäure), Treib- oder Inert-Gase (z.B. Fluorierte Chlorkohlenwasserstoffe, Kohlendioxid), Farbstoffe (Eisenoxide, Titandioxid), Salbengrundstoffe (z.B. Paraffine, Bienenwachs), u.a. wie sie in der Fachliteratur (z.B. Schmidt, P. C., Christin, I. "Wirk- und Hilfsstoffe für Rezeptur, Defektur und Großherstellung", 1999, Wissenschaftliche Verlagsgesellschaft mbH Stuttgart oder Bauer, K. H." Frömming, K-H., Führer, C. "Lehrbuch der Pharmazeutischen Technologie", 8. Auflage, 2006, Wissenschaftliche Verlagsgesellschaft mbH Stuttgart) vorkommen.

Die in einer pharmazeutischen Formulierung vorzugsweise einzusetzenden Mengen von einer oder mehreren Verbindungen der Formel (A) (wie vorstehend und nachfolgend, insbesondere in den Ansprüchen definiert) und/oder von einem oder mehreren von deren Salzen (wie vorstehend und nachfolgend, insbesondere in den Ansprüchen definiert) und/oder von entsprechenden Mischungen (wie vorstehend und nachfolgend, insbesondere in den Ansprüchen definiert) sowie von oben aufgeführten Bestandteilen können in Abhängigkeit von Art und Zweck der jeweiligen Formulierung vom Fachmann durch einfaches Ausprobieren leicht ermittelt werden.

Die vorliegende Erfindung betrifft auch eine Mischung umfassend eine oder mehrere Verbindungen der Formel (A) und/oder ein oder mehrere von deren Salzen, vorzugsweise ein oder mehrere pharmazeutisch akzeptable Salze der Verbindung der Formel (A) 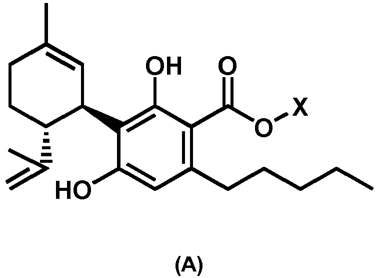 wobei X ein aliphatischer Rest mit einer, zwei, drei oder mehr als drei Hydroxygruppen ist, wobei die Gesamtzahl der C-Atome in dem aliphatischen Rest X nicht größer ist als 15, vorzugsweise nicht größer ist als 12,
und
wobei der aliphatische Rest
- gesättigt oder ungesättigt ist
   und
- verzweigt oder unverzweigt ist,
wobei in der Mischung
das molare Verhältnis der Gesamtmenge an Verbindungen der Formel (A) und deren Salzen, vorzugsweise pharmazeutisch akzeptablen Salzen, zur Menge an Cannabidiol (sofern vorhanden) größer ist als 1 : 1, bevorzugt größer ist als 5 : 1, besonders bevorzugt größer ist als 10 : 1
und gleichzeitig
das molare Verhältnis der Gesamtmenge an Verbindungen der Formel (A) und deren Salzen, vorzugsweise pharmazeutisch akzeptablen Salzen, zur Menge an der Verbindung der Formel (I) (sofern vorhanden) 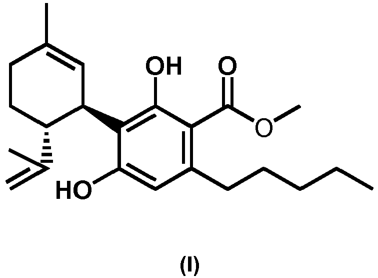 größer ist als 1 : 1, bevorzugt größer ist als 5 : 1, besonders bevorzugt größer ist als 10 : 1,
wobei die Verbindung der Formel (A) ausgewählt ist aus der Gruppe bestehend aus:

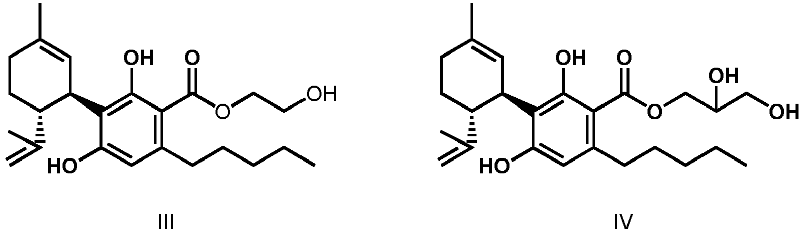

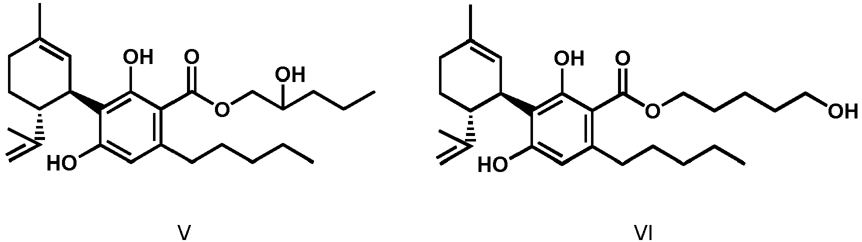

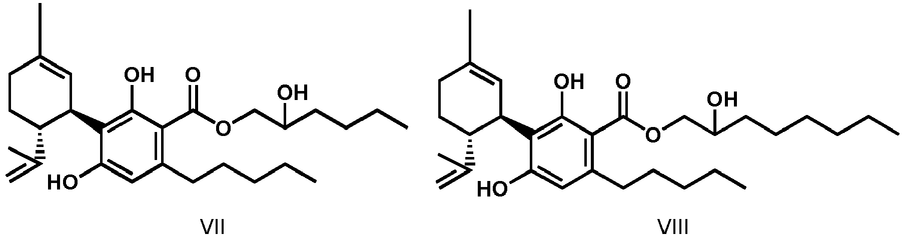

Sofern der aliphatische Rest X einer Verbindung der Formel (A) ein oder mehrere chirale Zentren besitzt, ist jede der möglichen Konfigurationen an dem bzw. jedem dieser Zentren gleichwertig (R oder S). Sofern im Einzelfall nicht anders angegeben, bezeichnet eine im vorliegenden Text zeichnerisch dargestellte individuelle Verbindung der Formel (A) mit einem oder mehreren chiralen Zentren im aliphatischen Rest sämtliche Konfigurationsisomere und sämtliche Mischungen von Konfigurationsisomeren der dargestellten Verbindung gleichermaßen, sofern diese durch Einstellung der Konfiguration an dem oder den chiralen Zentren des aliphatischen Restes darstellbar sind.

Je nach gewünschter Ausgestaltung und Zweck können auch erfindungsgemäße Mischungen (wie vorstehend und nachfolgend, insbesondere in den Ansprüchen definiert) einen oder mehrere der vorangehend im Zusammenhang mit erfindungsgemäßen pharmazeutischen Formulierungen genannten Bestandteile enthalten. Mischungen im Sinne der vorliegenden Erfindung können auch Halbfertigwaren zur Herstellung weiterer Verbindungen der Gruppe der Cannabinoide sein, die ihrerseits wiederum der Herstellung von pharmazeutischen Formulierungen dienen.

Aus einer Verbindung der Formel (A) lässt sich durch decarboxylierende Verseifung analog EP 2 314 580 A1 Cannabidiol herstellen. In einer erfindungsgemäßen Mischung überwiegt die Gesamtmenge an Verbindungen der Formel (A) und deren Salzen im Vergleich mit gegebenenfalls vorhandenem Cannabidiol.

Die Verbindung(en) der Formel (A) lassen sich durch Umesterung des Cannabidiolcarbonsäuremethylesters der Formel (I) herstellen; in einer erfindungsgemäßen Mischung überwiegt jedoch die Gesamtmenge an Verbindungen der Formel (A) und deren Salzen im Vergleich mit gegebenenfalls vorhandenem Methylester der Formel (I).

In erfindungsgemäßen Mischungen können demnach Cannabidiol und/oder Cannabidiolcarbonsäuremethylester (I) vorhanden sein, ihre Anwesenheit ist jedoch nicht verpflichtend.

Soweit eine erfindungsgemäße Mischung lediglich eine einzelne Verbindung der Formel (A) bzw. ein einzelnes Salz dieser einzelnen Verbindung der Formel (A) umfasst, enthält sie zumindest einen weiteren Bestandteil. Zu bevorzugten Bestandteilen siehe oben.

Eine erfindungsgemäße Mischung umfasst demnach beispielsweise (i) eine einzelne Verbindung oder (ii) ein einzelnes Salz oder (iii) mehrere Verbindungen oder (iv) mehrere Salze oder (v) eine Verbindung und ein Salz oder (vi) mehrere Verbindungen und ein oder mehrere Salze oder (vii) unterschiedliche Salze der gleichen Verbindung mit gleichem Deprotonierungsmuster (jedoch mit unterschiedlichen Kationen) oder (viii) im Deprotonierungsgrad sich unterscheidende Salze der gleichen Verbindung mit gleichen Kationen oder (ix) im Deprotonierungsgrad sich unterscheidende Salze der gleichen Verbindung jedoch mit gleicher oder unterschiedlichen Kationen oder (x) Salze unterschiedlicher Verbindungen mit gleichem Deprotonoierungsmuster und gleichen Kationen oder (xi) Salze unterschiedlicher Verbindungen mit unterschiedlichen Deprotonierungsmustern und gleichen oder unterschiedlichen Kationen oder (xii) Salze unterschiedlicher Verbindungen mit unterschiedlichem Deprotonierungsmuster und unterschiedlichen Kationen.

Bestimmte Verbindungen der Formel (A) (wie oben definiert) werden möglicherweise während des in EP 2314580 beschriebenen Verfahrens intermediär gebildet, jedoch würden dabei die Verbindung bzw. die Verbindungen der Formel (A) im Vergleich mit der Menge der Verbindung (I) und der Menge an Cannabidiol allenfalls in Spuren oder nur in geringen Mengen vorliegen.

Eine erfindungsgemäße Mischung ist vorzugsweise so zusammengesetzt, dass der Anteil der Gesamtmenge an Verbindungen der Formel (A) und deren Salzen in der Mischung bezogen auf das Gesamtgewicht der Mischung 0,0001 bis 100 Gew.-%, weiter bevorzugt 0,001 bis 100 Gew.-%, besonders bevorzugt 0,1 bis 100 Gew.-%, weiter bevorzugt von 1 bis 100 Gew.-% beträgt.

Für erfindungsgemäße Salze von Verbindungen der Formel (A) gilt: Gegebenenfalls liegen eine oder mehrere Hydroxygruppen einer Verbindung der Formel (A) deprotoniert vor. Neben der bzw. den (deprotonierten) Verbindung(en) der Formel (A) liegt dabei eine entsprechende Menge an Gegenkationen vor, wobei diese vorzugsweise ausgewählt sind aus der Gruppe bestehend aus: einfach positiv geladene Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, sowie Mischungen davon.

Die phenolischen Hydroxygruppen einer Verbindung der Formel (A) sind regelmäßig acider als Hydroxygruppen in der aliphatischen Seitenkette.

Aus der Zahl an deprotonierten Hydroxygruppen ergibt sich die entsprechende Anzahl an Gegenkationen (in Abhängigkeit von ihrer Ladung). So ergibt sich beispielsweise für eine einem solchen Salz zugrundeliegende Verbindung der Formel (A) mit zwei phenolischen Hydroxygruppen, dass bei vollständiger Deprotonierung dieser phenolischen Hydroxygruppen ein zweifach negativ geladenes Anion vorliegt, woraus sich wiederum die Zahl an positiven Ladungen ergibt (hier: zwei), die durch das bzw. die Gegenkation(en) bereitgestellt werden muss. Besonders bevorzugt handelt es sich bei diesen Gegenkationen um Kationen ausgewählt aus der Gruppe bestehend aus Na⁺, K⁺, NH₄⁺, Ca²⁺, Mg²⁺, Al³⁺ und Zn²⁺.

Bevorzugt ist eine erfindungsgemäße Mischung, wobei das molare Verhältnis der Gesamtmenge an Verbindungen der Formel (A) und deren Salzen zur Gesamtmenge an Verbindungen der Formel (II) 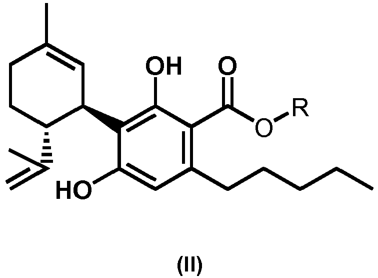 (d.h. Verbindungen der Formel (I) und sonstigen Verbindungen der Formel (II))
größer ist als 1 : 1, bevorzugt größer ist als 5 : 1, besonders bevorzugt größer ist als 10 : 1,
wobei R ausgewählt ist aus der Gruppe bestehend aus H und Schutzgruppen.

Der Begriff Schutzgruppen umfasst dabei sämtliche Gruppen, die gemäß EP 2314580 A1 als Schutzgruppen aufzufassen sind. Geeignet ist gemäß Abschnitt [0040] der EP 2314580 A1 für R eine Carboxyl-Schutzfunktion (Definition analog zu Herlt US-Patent 5,342,971 p. 4) von einem bis zu 16 Kohlenstoffatomen, typischerweise eine Alkylfunktion oder eine substituierte Alkylfunktion wie Benzyl (Phenylmethyl-), Diphenylmethyl- oder solchen in 2-Stellung substituierten Alkylresten von einem bis 16 C-Atomen wie (i) niederem Alkoxy-z.B. 2-Methoxyethyl , 2-Ethoxyethyl, (ii) niederem Alkylthio wie z. B. 2-Methylthioethyl-und 2-Ethylthioethyl, (iii) Halogen wie 2,2,2-Trichlorethyl, 2-Bromethyl und 2-Chlorethyl, (iv) einer oder zwei Phenylgruppen (substituiert oder unsubstituiert) substituierten Alkylgruppen; sowie Aroylgruppen wie Phenacyl. Die in Verbindungen der Formel (A) enthaltenen aliphatischen Reste mit einer, zwei, drei oder mehr als drei Hydroxygruppen, wobei die Gesamtzahl der C-Atome in dem aliphatischen Rest X nicht größer ist als 15, werden nicht als Schutzgruppe aufgefasst.

Auch in bevorzugten erfindungsgemäßen Mischungen können demnach Cannabidiol (R= H) und/oder Cannabidiolcarbonsäuremethylester (I) (R = Me) und/oder weitere Verbindungen der Formel (II) wie oben definiert vorhanden sein, ihre Anwesenheit ist jedoch weder verpflichtend noch soll ihre Gesamtmenge größer sein als die Gesamtmenge an Verbindungen der Formel (A) und deren Salzen. Als besonders vorteilhaft bezüglich ihrer (wie oben oder unten beschriebenen) Eigenschaften und/oder ihres Einsatzes in erfindungsgemäßen Verfahren haben sich erfindungsgemäße Mischungen erwiesen, in denen das molare Verhältnis der Gesamtmenge an Verbindungen der Formel (A) und deren Salzen zur Gesamtmenge an Verbindungen der Formel (II) größer ist als 5 : 1, besonders bevorzugt größer ist als 10 : 1, da auf diese Weise regelmäßig Konkurrenzreaktionen der oben beschriebenen Verbindungen der Formel (II) mit Verbindungen der Formel (A) um CB1- bzw. CB2-Rezeptorstellen zurückgedrängt als auch erhebliche Ausbeuteverluste bei nachfolgenden Umsetzungen erfindungsgemäßer Mischungen in erfindungsgemäßen Verfahren vermieden werden.

Bevorzugt ist eine erfindungsgemäße Mischung (wie vorstehend oder nachfolgend, insbesondere in den Ansprüchen definiert), wobei die Zahl der Hydroxygruppen in dem aliphatischen Rest X eins, zwei oder drei ist, vorzugsweise eins oder zwei.

Als besonders vorteilhaft haben sich in eigenen Untersuchungen erfindungsgemäße Mischungen erwiesen, deren Verbindungen der Formel (A) bzw. deren Salze im aliphatischen Rest X eine, zwei oder drei, vorzugsweise eine oder zwei Hydroxygruppen tragen, Diese Verbindungen besitzen zwar durch die Anwesenheit der besagten ein, zwei oder drei, vorzugsweise ein oder zwei Hydroxygruppen im aliphatischen Rest die Löslichkeit, welche für die vorstehend oder nachfolgend beschriebenen Anwendungen bzw. Reaktionen erforderlich ist, jedoch besitzen sie keine derart hohe Anzahl an aliphatischen Hydroxygruppen, dass unerwünschte Nebenreaktionen wie z.B. Eliminierungsreaktionen in störendem Maße auftreten.

Als besonders vorteilhaft haben sich erfindungsgemäße Mischungen erwiesen, wobei der aliphatische Rest der Verbindung der Formel (A) gesättigt und unverzweigt ist, da ungesättigte aliphatische Reste das Risiko von unerwünschten Nebenreaktionen erhöhen und verzweigte aliphatische Reste die sterischen Anforderungen für erfindungsgemäße Mischungen in der Regel nicht in gleichem Maße erfüllen (v.a. für die Anwendung als Arzneimittel oder die Anwendung in einem Verfahren zur therapeutischen Behandlung des menschlichen oder tierischen Körpers).

Erfindungsgemäß ist die Verbindung der Formel (A) ausgewählt aus der Gruppe bestehend aus:

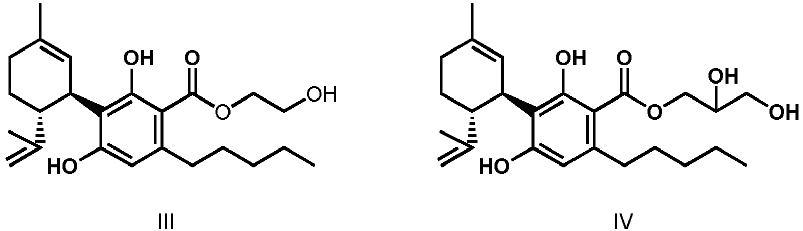

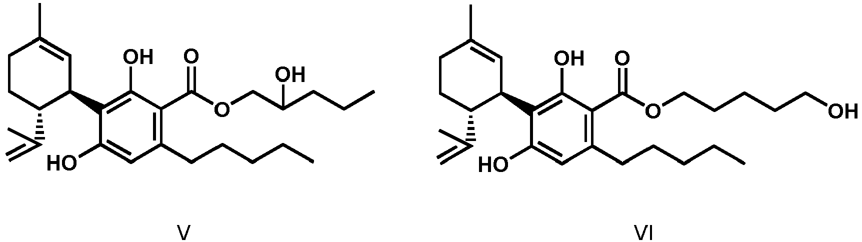

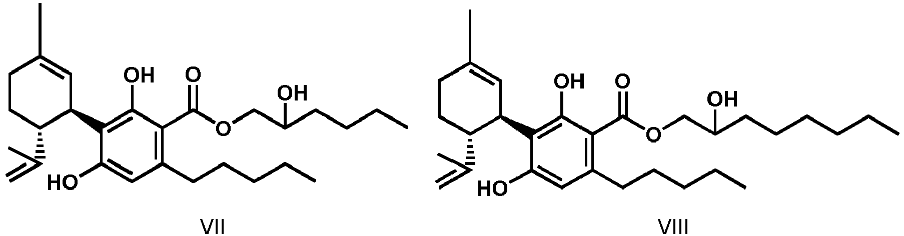

Zu den Verbindungen III bis V sei auf die weiter oben und in den Beispielen angegebenen Eigenschaften und Vorteile verwiesen; sehr ähnliche Eigenschaften und Vorteile bestehen auch für die Verbindungen VI bis VIII, die Ausführungen betreffend die Verbindungen III bis V gelten mutatis mutandis. Selbstverständlich werden auch die Salze der Verbindungen III-VIII (wie vorstehend und nachfolgend, insbesondere in den Ansprüchen definiert) bevorzugt eingesetzt.

Die Verbindungen III-VIII sind durch die Formeln A-I, A-II, A-III, A-IV wie folgt definiert.

| **Formel** | | **Verbindung III** | **Verbindung IV** | **Verbindung V** |
|---|---|---|---|---|
| (A) | X | OCH₂CH₂OH | OCH₂CH(OH)CH₂OH | OCH₂CH(OH)CH₂CH₂CH₃ |
| (A-I) | 1. R¹ | H | H | H |
| | 2. R¹ | H | OH | OH |
| | 3. R¹ | / | H | H |
| | 4. R¹ | / | / | H |
| | 5. R¹ | / | / | / |
| | 6. R¹ | / | / | / |
| | 7. R¹ | / | / | / |
| | R² | OH | OH | CH₃ |
| | n | 2 | 3 | 4 |
| (A-II) | 1. R¹ | H | OH | OH |
| | 2. R¹ | / | H | H |
| | 3. R¹ | / | / | H |
| | 4. R¹ | / | / | / |
| | 5. R¹ | / | / | / |
| | 6. R¹ | / | / | / |
| | R² | OH | OH | CH₃ |
| | n | 2 | 3 | 4 |
| (A-III) | 1. R¹ | / | H | H |
| | 2. R¹ | / | / | H |
| | 3. R¹ | / | / | / |
| | 4. R¹ | / | / | / |
| | 5. R¹ | / | / | / |
| | R² | / | OH | CH₃ |
| | n | / | 3 | 4 |
| (A-IV) | 1. R¹ | H | OH | / |
| | 2. R¹ | / | H | / |
| | 3. R¹ | / | / | / |
| | 4. R¹ | / | / | / |
| | n | 2 | 3 | / |
| | | | | |

| **Formel** | | **Verbindung VI** | **Verbindung VI** | **Verbindung VIII** |
|---|---|---|---|---|
| (A) | X | O(CH₂)₅OH | OCH₂CH(OH)CH₂(CH₂)₂CH₃ | OCH₂CH(OH)CH₂(CH₂)₄CH₃ |
| (A-I) | 1. R¹ | H | H | H |
| | 2. R¹ | H | OH | OH |
| | 3. R¹ | H | H | H |
| | 4. R¹ | H | H | H |
| | 5. R¹ | H | H | H |
| | 6. R¹ | / | / | H |
| | 7. R¹ | / | / | H |
| | R² | OH | CH₃ | OH |
| | n | 5 | 5 | 7 |
| (A-II) | 1. R¹ | H | OH | OH |
| | 2. R¹ | H | H | H |
| | 3. R¹ | H | H | H |
| | 4. R¹ | H | H | H |
| | 5. R¹ | / | / | H |
| | 6. R¹ | / | / | H |
| | R² | OH | CH₃ | CH₃ |
| | n | 5 | 5 | 7 |
| (A-III) | 1. R¹ | / | H | H |
| | 2. R¹ | / | H | H |
| | 3. R¹ | / | H | H |
| | 4. R¹ | / | / | H |
| | 5. R¹ | / | / | H |
| | R² | / | CH₃ | CH₃ |
| | n | / | 5 | 7 |
| (A-IV) | 1. R¹ | H | / | / |
| | 2. R¹ | H | / | / |
| | 3. R¹ | H | / | / |
| | 4. R¹ | H | / | / |
| | n | 5 | / | / |

Die Erfindung betrifft auch ein Verfahren zur Herstellung einer erfindungsgemäßen Mischung (wie vorstehend oder nachfolgend, insbesondere in den Ansprüchen definiert), mit folgendem Schritt:

Umsetzen eines Cannabidiolcarbonsäureesters der Formel (IX) 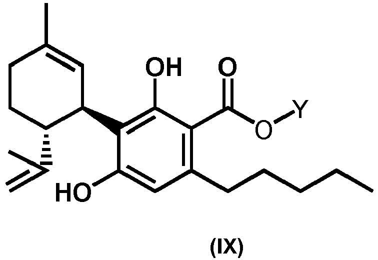 wobei Y ein organischer Rest ist,
mit einem Alkohol der Formel HO-X,
wobei
X ein aliphatischer Rest mit einer, zwei, drei oder mehr als drei Hydroxygruppen ist, wobei die Gesamtzahl der C-Atome in dem aliphatischen Rest X nicht größer ist als 15, vorzugsweise nicht größer ist als 12, und
wobei der aliphatische Rest
- gesättigt oder ungesättigt ist
   und
- verzweigt oder unverzweigt ist,
wobei Y verschieden von X ist und so gewählt ist, dass bei der Umsetzung entstehender Alkohol der Formel HO-Y bei 1013 hPa bei niedrigerer Temperatur siedet als der eingesetzte Alkohol der Formel HO-X.

Das Produkt des erfindungsgemäßen Verfahrens ist eine erfindungsgemäße Mischung.

Bei der Umsetzung eines Cannabidiolcarbonsäureesters der Formel (IX) mit Alkali in hochsiedenden Lösungsmitteln der Formel HO-X ohne Anwesenheit von Wasser wurde überraschenderweise festgestellt, dass diese Umsetzung nicht direkt zur Cannabidiolcarbonsäure führt, sondern zum entsprechenden Umesterungsprodukt, d.h. einer Verbindung der Formel (A). Diese Verbindung konnte aus dem Reaktionsgemisch in hoher Ausbeute isoliert werden. Neben ihrer Eigenschaft als CB1-/CB2-Rezeptoren können diese Verbindungen der Formel (A) überdies dazu verwendet werden, Cannabidiol bzw. Delta-9-THC herzustellen.

Im Gegensatz hierzu beschreibt EP 2314580 A1 die Verseifung der Verbindung (I) zu Cannabidiol durch Behandlung mit Alkali in einem Gemisch aus Methanol/Wasser, wobei die Reaktion unter Druck bei 140-150°C durchgeführt wird. Alternativ kann die Reaktion "drucklos" unter Verwendung eines "mit Wasser mischbaren Lösungsmittel mit einem Siedepunkt über 100°C bei Normaldruck" durchgeführt werden. Verbindungen der Formel (A) (wie oben definiert) werden dabei gemäß EP 2314580 A1 nicht identifiziert und nicht isoliert.

Bisherige Verfahren zur Herstellung von Cannabidiol oder cannabinoiden Verbindungen, die folgende Schritte umfassen:
a) Kupplung eines geeigneten Terpens mit einem Resorcin-Derivat (Schritt I),
b) Verseifung und Decarboxylierung der Estergruppe im Resorcin-Derivat (Schritt II) und
c) Cyclisierung des Intermediats zu Cannabidiol (Schritt III) beinhalten folgende Nachteile bei der angegebenen Verfahrensweise, insbesondere in Hinsicht auf eine technische Herstellung:
   - Kühlung der Reaktion durch tiefe Temperaturen (Schritt-I), und lange Reaktionsdauer, was die Wirtschaftlichkeit des Verfahrens in Form des Energieverbrauches einerseits und der relativ langen Arbeitszeit andererseits, negativ beeinflusst,
   - Verwendung von leicht-flüchtigem Methylenchlorid (Schritt-I und III), welches als gesundheitsschädlich und potentiell karzinogen eingestuft wird und durch die notwendig hohen Sicherheitsvorkehrungen beim Umgang mittelbar ebenfalls die Wirtschaftlichkeit und die ökologische Verträglichkeit des Verfahrens negativ beeinflusst sowie
   - hohe Verdünnung und damit schlechte Raum-Zeit-Ausbeute (Schritt-I und III).

Besonders bevorzugt ist ein erfindungsgemäßes Verfahren zur Herstellung einer erfindungsgemäßen Mischung, wobei Y eine Alkylgruppe ist, die vorzugsweise ausgewählt ist aus der Gruppe bestehend aus Methyl, Ethyl, n-Propyl, iso-Propyl, n-Butyl, sec-Butyl, iso-Butyl, tert-Butyl.

Die Alkylgruppen Methyl, Ethyl, n-Propyl, iso-Propyl, n-Butyl, sec-Butyl, iso-Butyl und tert-Butyl haben sich als vorteilhafte Reste Y bei der Umsetzung eines Cannabidiolcarbonsäureesters der Formel (IX) mit einem Alkohol der Formel HO-X, herausgestellt; ihre korrespondierenden Alkohole lassen sich unter den nachfolgend beschriebenen Reaktionsbedingungen effektiv aus dem Reaktionsgemisch entfernen, was regelmäßig sowohl zu einer besonders guten Ausbeute führt als auch die Anforderungen an den Reaktionsaufbau vereinfacht.

Ganz besonders bevorzugt ist ein erfindungsgemäßes Verfahren zur Herstellung einer erfindungsgemäßen Mischung, wobei das Umsetzen des Cannabidiolcarbonsäureesters der Formel (IX) mit dem Alkohol der Formel HO-X bei einem Druck erfolgt, der geringer ist als 1013 hPa, vorzugsweise bei einem Druck im Bereich von **5** bis **500** hPa.

Es ist insbesondere deshalb vorteilhaft, die Reaktion nicht bei Normaldruck, sondern unter Vakuum durchzuführen, da dies eine effiziente Entfernung des entstehenden Alkohols der Formel HO-Y (z.B. Methanol, Ethanol, n-Propanol, iso-Propanol, n-Butanol, sec-Butanol, iso-Butanol, tert-Butanol) aus dem Reaktionsgemisch ermöglicht und damit den Fortgang der Reaktion begünstigt. Der durch Umesterung entstehende Alkohol der Formel HO-Y wird vorzugsweise mittels Destillation aus dem Reaktionsgemisch entfernt.

Besonders bevorzugt ist ein erfindungsgemäßes Verfahren zur Herstellung einer erfindungsgemäßen Mischung mit folgendem Schritt zur Herstellung des Cannabidiolcarbonsäureesters der Formel (IX):
- Umsetzen von Menthadienol mit einem Olivetolcarbonsäureester zum entsprechenden Cannabidiolcarbonsäureester der Formel (IX) in einem kontinuierlichen Verfahren.

Es wurde nämlich überraschend festgestellt, dass die Umsetzung von Menthadienol mit einem Olivetolcarbonsäureester zum entsprechenden Cannabidiolcarbonsäureester der Formel (IX) mit einer sehr hohen Reaktionsgeschwindigkeit abläuft, so dass das Verfahren in einer kontinuierlichen Verfahrensweise mit hoher Raum-Zeit-Ausbeute durchführbar ist. Im Rahmen der entsprechenden Untersuchung wurde eine Lösung der beiden Ausgangsprodukte mit einer Lösung eines Lewis-Säure-Katalysators kontinuierlich in eine gerührte Reaktionszelle gepumpt und anschließend in eine gesättigte wässrige Natriumbicarbonatlösung geleitet, um den Katalysator zu hydrolysieren und eine Weiterreaktion zu Nebenprodukten zu verhindern.

Die Umsetzung von Menthadienol mit einem Olivetolcarbonsäureester kann in verschiedenen Lösungsmitteln durchgeführt werden, wie z.B. Methylenchlorid, Chlorbenzol, Toluol, Xylol und Cyclohexan, wobei Methylenchlorid und Chlorbenzol deutlich bessere Ausbeuten zeigen, aus gewerbe-hygienischen Gründen jedoch hochsiedendes Chlorbenzol zu favorisieren ist.

Als Katalysatoren sind (Lewis)-Säuren wie Bortrifluorid*Etherat, Bortrifluorid*Essigsäure, Titantetrachlorid, p-Toluolsulfonsäure oder Methansulfonsäure verwendbar, wobei Bortrifluorid*Etherat besonders gute Ergebnisse erzielt.

Die Erfindung betrifft auch ein Verfahren zur Herstellung von Delta-9-Tetrahydrocannabinol, umfassend die Herstellung einer erfindungsgemäßen Mischung, wobei die erfindungsgemäße Mischung vorzugsweise mittels eines erfindungsgemäßen Verfahrens (wie oben definiert) hergestellt wird.

Es ist besonders vorteilhaft, Delta-9-Tetrahydrocannabinol ausgehend von einer erfindungsgemäßen Mischung zu synthetisieren (wobei diese Mischung vorzugsweise mittels eines erfindungsgemäßen Verfahrens (wie oben definiert) hergestellt wird), da die intermediär hergestellte erfindungsgemäße Mischung sowie die üblicherweise im Anschluss intermediär gebildete Verbindung Cannabidiol (X) selbst individuelle biologische Aktivitäten aufweisen und somit in bestimmtem Ausmaß aus dem Verfahren abgetrennt werden können, um selbst als cannabinoide Wirksubstanzen eingesetzt zu werden. Zudem ist eine Unterbrechung des Verfahrens, eine Lagerung der intermediär hergestellten erfindungsgemäßen Mischungen und eine spätere Fortsetzung der Synthese an gleichem oder anderem Ort vorteilhafterweise möglich.

Ganz besonders bevorzugt ist ein erfindungsgemäßes Verfahren zur Herstellung von Delta-9-Tetrahydrocannabinol, umfassend die Herstellung einer erfindungsgemäßen Mischung umfassend eine oder mehrere Verbindungen der Formel (A) und/oder ein oder mehrere von deren Salzen, vorzugsweise ein oder mehrere pharmazeutisch akzeptable Salze der Verbindung der Formel (A), 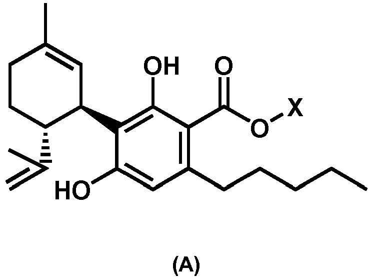 wobei X ein aliphatischer Rest mit einer, zwei, drei oder mehr als drei Hydroxygruppen ist, wobei die Gesamtzahl der C-Atome in dem aliphatischen Rest X nicht größer ist als 15, vorzugsweise nicht größer ist als 12, und
wobei der aliphatische Rest
- gesättigt oder ungesättigt ist
   und
- verzweigt oder unverzweigt ist,
wobei in der Mischung
das molare Verhältnis der Gesamtmenge an Verbindungen der Formel (A) und deren Salzen, vorzugsweise pharmazeutisch akzeptablen Salzen, zur Menge an Cannabidiol (sofern vorhanden) größer ist als 1 : 1, bevorzugt größer ist als 5 : 1, besonders bevorzugt größer ist als 10 : 1
und gleichzeitig
das molare Verhältnis der Gesamtmenge an Verbindungen der Formel (A) und deren Salzen, vorzugsweise pharmazeutisch akzeptablen Salzen zur Menge an der Verbindung der Formel (I) (sofern vorhanden) 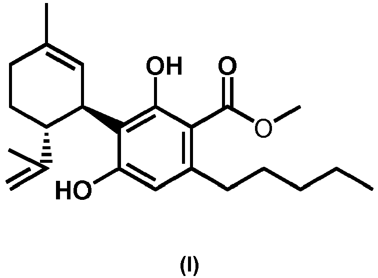 größer ist als 1 : 1, bevorzugt größer ist als 5 : 1, besonders bevorzugt größer ist als 10 : 1,
wobei die erfindungsgemäße Mischung gemäß einem erfindungsgemäßen Verfahren mit folgendem Schritt hergestellt wird:

Umsetzen eines Cannabidiolcarbonsäureesters der Formel (IX) 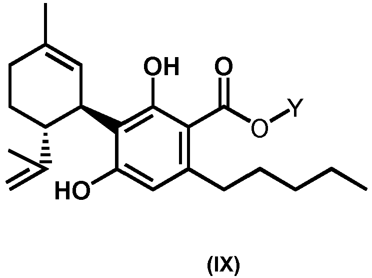 wobei Y ein organischer Rest ist,
mit einem Alkohol der Formel HO-X,
wobei
X die oben angegebene Bedeutung hat und
wobei Y verschieden von X ist und so gewählt ist, dass bei der Umsetzung entstehender Alkohol der Formel HO-Y bei 1013 hPa bei niedrigerer Temperatur siedet als der eingesetzte Alkohol der Formel HO-X.

Besonders bevorzugt ist ein erfindungsgemäßes Verfahren zur Herstellung von Delta-9-Tetrahydrocannabinol, wobei die hergestellte erfindungsgemäße Mischung so behandelt wird, dass die in der Mischung enthaltene Verbindung der Formel (A) decarboxylierend verseift und die Verbindung (X) (Cannabidiol) gebildet wird.

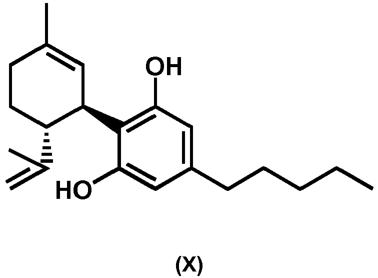

Ganz besonders bevorzugt ist ein erfindungsgemäßes Verfahren zur Herstellung von Delta-9-Tetrahydrocannabinol, wobei die nach decarboxylierender Verseifung vorliegende Verbindung (X) zu Delta-9-Tetrahydrocannabinol cyclisiert wird, vorzugsweise in Abwesenheit halogenhaltiger Lösungsmittel.

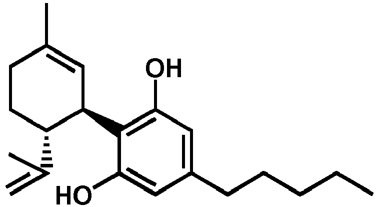

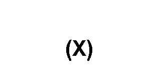

Überraschenderweise konnte das im Stand der Technik bei der Cyclisierung verwendete chlorhaltige Methylenchlorid in eigenen Untersuchungen ohne Nachteile durch den unbedenklichen Methyl-tert.-butylester ersetzt werden. Dies gelang auch bei Konzentrationen von bis zu 20 Gew.-% Cannabidiol im Ausgangsgemisch.

Nachfolgend wird die vorliegende Erfindung anhand von Beispielen näher erläutert.

### A. Untersuchungen zur Wirkung erfindungsgemäßer Verbindungen an Cannabinoid-Rezeptoren:

In eigenen Untersuchungen wurden insbesondere die folgenden Stoffe III-V der allgemeinen Formel (A) auf ihre Wirkung an Cannabinoid-Rezeptoren untersucht.

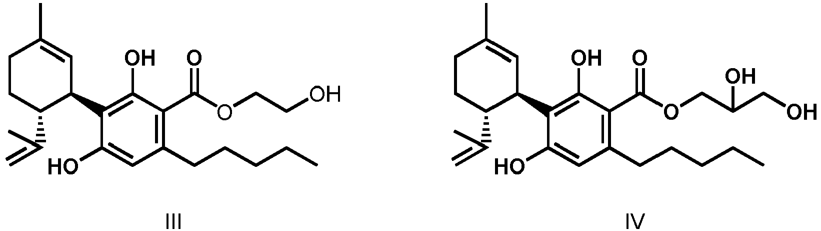

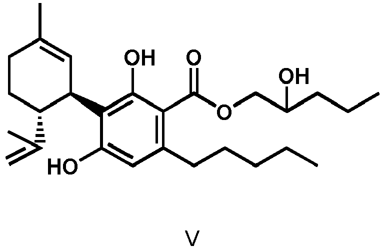

Die Stoffe III-V wurden in Kompetitions-Studien auf Ihre Bindungsaffinität und ihr resultierendes Bindungsprofil zu CB1- und CB2-Rezeptoren untersucht. Solche Studien erlauben es, die Affinität eines jeden der Stoffe III-V (Kᵢ-Werte) mit der eines anderen Liganden von Cannabinoid-Rezeptoren zu vergleichen. Die Kompetitions-Studien wurden in Membranen von Zellen durchgeführt, die mit CB1- oder CB2-Rezeptoren transfiziert wurden. Dazu wurden Membranen vom menschlichen Zellen verwendet, in die CB1- oder CB2-Rezeptoren (RBHCB1M400UA und RBXCB2M400UA) mit einem Bₘₐₓ- und K_{d}-Wert für CP55940 für CB1 oder CB2 von beispielsweise 1,9 pmol/mg Membran Protein und 0,18 nM für CB1 und 5,2 pmol/mg Membran Protein und 0,18 nM für CB2 transfiziert wurden.

In einem beispielhaften Experiment lag die Protein-Konzentration der CB1-rezeptortragenden Membranen bei 8,0 mg/ml und die der CB2-rezeptortragenden Membranen bei 4,0 mg/ml. Diese und die weiteren Werte ergaben sich aus den Angaben des Herstellers der Membranen und sind durch den kundigen Fachmann genauso leicht nachvollziehbar, wie die Techniken, mit denen die Studien durchgeführt wurden. Die Membran-Suspensionen wurden in einer Verdünnung von 1:20 mit Pufferlösung (50 nM TrisCl, 5 nM MgCl₂,xH₂O, 2,5 nM EDTA, 0,5 mg/ml BSA und pH 7,4 für CB1 Bindungs-Puffer; 50 nM TrisCl, 5 nM MgCl₂,xH₂O, 2,5 nM EGTA, 1 mg/ml BSA und pH 7,5 für CB2 Bindungs-Puffer) verdünnt. Als Radioligand wurde [³H]-CP55940 (144 Ci/mmol) eingesetzt. Beispielhafte Konzentrationen waren hierbei 0,10 nM mit einem Volumen von 200 µl für CB1 Bindungsstudien und 0,15 nM mit einem finalen Volumen von 600 µl für CB2 Bindungsstudien. Die Membranen wurden im Puffer resuspendiert, mit dem Radioliganden und mit jeder Substanz für 90 min bei 30 °C inkubiert. Unspezifische Bindung wurde mit Hilfe des klassischen Liganden WIN55212-2 bestimmt und die 100 %-ige Bindung des Radioliganden wurde dadurch bestimmt, dass die Membran ohne eine andere Substanz inkubiert wurde. Nach der Filtration des jeweiligen Ansatzes wurde neun Mal mit dem jeweiligen Bindungs-Puffer gewaschen und anschließend getrocknet. Die Radioaktivität wurde mit einem geeigneten Szintillationszähler bestimmt. Entsprechende Modelle sind bereits in der Literatur bekannt (Granja, A. G. et al. J. Neuroimmune Pharmacol. 2012, 7, 1002-1016; Cumella, J. et al. ChemMedChem. 2012, 7, 452-463; Di Marzo, V. et al. 2000, J. Neurochem., 2000, 74, 1627-1635).

Eine Bewertung der Komponenten wurde in zwei Phasen vollzogen. Die erste Phase bestand aus einem screening mit einer einfachen hohen Dosis jeder Substanz auf deren Bindungsfähigkeit. Die folgende Tabelle gibt die prozentualen Werte für die Bindung an CB1 und CB2 wieder:

**Tabelle 1: Prozentuale Bindung ausgewählter Cannabinoide an Cannabinoid-Rezeptoren**

| **Substanz** | **CB1 (% Bindung)** | **CB2 (% Bindung)** |
|---|---|---|
| **III** | 77.3 ± 6.5 | 90.3 ± 2.4 |
| **IV** | 90.2 ± 3.8 | **101.6** ± 0.5 |
| **V** | 82.0 ± 8.0 | 83.9 ± 5.6 |

Anmerkung: Der Messwert von 101.6± 0.5 beruht auf einer üblichen, wissenschaftlich akzeptierten Messungenauigkeit des verwendeten Modells.

Substanzen, die über 50 % Bindung und damit Verdrängung von [³H]-CP55940 zeigen, wurden in einer zweiten Phase auf Ihre Kompetition um CB1 und CB2 getestet, indem man verschiedene Konzentrationen der Substanzen zusammen mit [³H]-CP55940 in dem Rezeptormodell inkubierte. Die daraus resultieren Daten wurden mit Hilfe einer geeigneten Statistik Software (z.B. GraphPrism^{®} Version 5.01) ausgewertet. Die folgende Tabelle gibt die Dissoziationskonstanten (Kᵢ) für die Substanzen als Mittelwert +/- Standardfehler (SEM) an:

**Tabelle 2: Dissoziationskonstanten der cannabinoiden Verbindungen III-V**

| **Substanz** | **Kᵢ für CB1 (nM)** | **Kᵢ für CB2 (nM)** | **Kᵢ (CB1) : Kᵢ (CB2)** |
|---|---|---|---|
| | | | **Selektivität für CB2 im Vergleich zu CB1 (gerundet)** |
| **III** | 3923 ± 1547 | 374.5 ± 47.7 | 10.4 |
| **IV** | 2174 ± 1149 | 277.1 ± 78.7 | 7.8 |
| **V** | 538.2 ± 53.9 | 66.7 ± 13.1 | 8.1 |

Im Vergleich dazu zeigte die Substanz WIN55,212-2, die als klassischer nicht spezifischer Ligand als positiv Kontrolle für ein solches Experiment verwendet wurde, eine Dissoziationskonstante von 28,8 +/41 nM für CB1 und 3,7 +/-1 nM für CB2 und entspricht damit den literaturverzeichneten Werten (z.B. Pertwee, R. G. et al. Pharmacol. Rev. 2010, 62, 588-631).

Fazit / vergleichende Bewertung:

Die cannabinoiden Substanzen III-V binden in nM-Konzentrationen und damit in physiologischen Dosen an Cannabinoid-Rezeptoren. Sie sind schwache Liganden an CB1-Rezeptoren und binden bevorzugt an CB2-Rezeptoren. Ihre Selektivität für CB2-Rezeptoren prädestiniert sie für den Einsatz als CB2-Rezeptormodulatoren.

Literaturbekannte Cannabinoide und Substanzen, die nicht zu den klassischen Cannabinoiden zu zählen sind, werden aufgrund ihrer Affinität zu CB1- bzw. CB2-Rezeptoren in Gruppen eingeteilt (Pertwee, R. G. et al. Pharmacol. Rev. 2010, 62, 588-631). Die Gruppenzugehörigkeit und damit der pharmakodynamische Mechanismus determiniert die Art und Weise der Wirkung der Substanzen.

Während CBD mit sehr geringen Affinitäten (CB1: 4.350 bis >10.000 nM; CB2: 2.399 bis >10.000 nm), eine sehr schwache Wirkung ausübt, ist Delta-9-THC mit CB1: 5,05 bis 80,3 nM und CB2: 3.13 bis 75.3 nM ein starker Ligand an beiden Rezeptoren, was auch seine starken zentralnervösen Wirkungen (auch Nebenwirkungen) und gleichzeitigen peripheren Wirkungen (und Nebenwirkungen) erklärt. Die psychotropen Effekte von Delta-9-THC werden seiner komplexen Interaktion mit dem CB1-Rezeptor zugeschrieben. Die Aktivierung des CB1-Rezeptors verursacht unerwünschte Wirkungen auf die Psyche (und den Kreislauf), die Aktivierung des CB2-Rezeptors hingegen scheint dies nicht zu tun, was auch an der Lokalisation der CB2-Rezeptoren in der Peripherie liegt (Atwood, B. K. Prog. Neuropsychopharmacol. Biol. Psychiatry 2012, 38, 16-20).

Die hier beschriebenen Cannabinoide haben eine günstige und einzigartige Verteilung ihrer Bindungsaffinität (siehe Tabelle 2). Ihrer Bindungsaffinität hin zu einer attenuierten, jedoch nicht vollständig aufgehobenen Ansprache des CB1-Rezeptors prädestiniert die Substanzen als Pharmaka. Die Nachweise für eine vorteilhafte Wirkung von CB2-Modulatoren in bisher nicht der Pharmakotherapie zugänglichen pathologischen Situationen sind in den letzten Jahren stark gewachsen. Die beiden wichtigsten Indikationen für CB2-Modulatoren sind Neuroinflammation und Schmerzen (Cheng, Y., Hitchcock, S. A. Expert Opin. Invest. Drugs 2007, 16, 951-965; Guindon, J., Hohmann, A. G. J. Pharmacol. 2008, 153, 319-334). Weiterhin können erfindungsgemäße Substanzen aber auch durch CB2-Modulation folgende pathologische Situationen beeinflussen: Systemische Inflammation, Osteoporose, Krebs, Transplantationsbedingte pathologische Zustände, verschiedene pathologische Zustände des zentralen Nervensystems einschließlich Drogenabhängigkeit und Angstzustände sowie Erkrankungen der Leber (Bab, I. et al. Ann. Med. 2009, 41, 560-567; Karsak, M. et al. Science 2007, 316, 1494-1497; Mallat, A., Lotersztajn, S. Dig. Dis. 2010, 28, 261-266; Nagarkatti, M. et al. Trends Pharmacol. Sci. 2010, 31, 345-350; Patel, K. D. et al. Curr. Med. Chem. 2010, 17, 1393-1410; Xi, Z. X. et al. Nat. Neurosci. 2011, 14, 1160-1166).

### B. Synthese von Delta-9-THC via 2-Hydroxyethylcannabidiolat (III)

### Schritt 1: Kupplungsschritt im kontinuierlichen Verfahren); Synthese von Cannabidiol-carbonsäuremethylester (I)

300g (2,0 Mol) Menthadienol und 476g (2,0 Mol) Olivetolester werden bei circa 22°C in 1.370g Chlorbenzol gelöst (2.000mL Lösung A), desgleichen werden 94g (0,66 Mol) Bortrifluorid*Etherat in 640g Chlorbenzol bei circa 22°C gelöst (666mL Lösung B). Über zwei getrennte Dosierpumpen werden dann Lösung A mit einem Fluss von 72mL/min und Lösung B mit einem Fluss von 24mL/min in eine gerührte Reaktionszelle gepumpt, aus der Reaktorzelle läuft das Reaktionsgemisch über einen PTFE-Schlauch in eine gerührte 1.000g Natriumbicarbonat-Lösung. Die Gesamtreaktionsdauer beträgt ca. 20min. Nach Ende der Dosierung wird die hydrolysierte Reaktionslösung noch ca. 30min nachgerührt.

Man überträgt dann die hydrolysierte Reaktionslösung in einen 5-Ltr.-Doppelmantel-Reaktionsgefäß trennt die wässrige Phase - und destilliert das Lösungsmittel Chlorbenzol ab. Man gibt ca. 2.000g Toluol zu dem verbleibenden 730g Rohmaterial und extrahiert den nicht umgesetzten Olivetolester durch viermalige Zugabe von 1.200g 1 %-iger wässriger Natronlauge. Nach Ansäuern mit halb-konz. Schwefelsäure und Re-Extraktion dieser wässrigen Phase gewinnt man ca. 30% (140g) nicht umgesetzten Olivetolester zurück.

In der Toluolphase befinden sich ca. 520g Cannabidiolcarbonsäuremethylester (I), was einer Ausbeute von ca. 70% d.Th. entspricht. Dieses erste Zwischenprodukt dient als Ausgangsverbindung für die anschließende Umesterung.

### Schritt 2: Umesterung, Synthese von 2-Hydroxyethylcannabidiolat (III):

Das Toluol wird destillativ entfernt und zum verbleibenden ersten Zwischenprodukt gibt man unter Rühren 600g Ethylenglykol und versetzt mit einer Lösung aus 85g Kaliumhydroxid in 300g Ethylenglykol. Man legt ein Vakuum von ca. 0,5 bar an, und erhitzt auf 120°C für 2h, wobei ca. 40g Methanol abdestillieren. Das resultierende Produktgemisch umfasst hauptsächlich 2-Hydroxyethylcannabidiolat (III).

### Schritt 3: Verseifung / Decarboxylierung, Synthese von Cannabidiol (X):

Danach erhöht man die Temperatur auf 150°C und rührt bei dieser Temperatur für 2h. Man kühlt das aus der Umesterung resultierende Produktgemisch umfassend hauptsächlich 2-Hydroxyethylcannabidiolat (III) auf ca. 40°C ab und versetzt mit 500g Wasser sowie mit 500g n-Heptan und gibt zur Neutralisation ca. 150g halbkonz. Schwefelsäure hinzu. Nach Phasentrennung wird das Lösungsmittel abrotiert und der Rückstand über einen Dünnschichtverdampfer bei einem Vakuum von ca. 0,5 mbar und einer Manteltemperatur von 230°C destilliert. Man erhält 310g Cannabidiol (X) in Form eines viskosen, gelblichen Öls mit einer Reinheit von 85%, das entspricht einer Ausbeute von 60% d.Th. bezogen auf eingesetzten Cannabidiolcarbonsäureester.

Dieses visköse, gelbliche Öl wird dann in ca. 200g n-Heptan bei ca. -5°C umkristallisiert, wonach 210g weißes Kristallisat mit einer Reinheit von 99% Cannabidiol (X) vorliegt.

### Schritt 4: Cyclisierung, Synthese von Delta-9-THC:

50g reines Cannabidiol werden in 250g Methyl-tert.-butylether gelöst und bei circa 22°C innerhalb von 10min mit 40g Bortrifluorid*Essigsäure-Komplex unter Rühren versetzt. Man rührt 3h bei genannter Temperatur und gibt dann 200g Eiswasser hinzu, wäscht die organische Phase mit Natriumbicarbonatlösung und rotiert das Lösungsmittel ab. Das verbleibende Rohmaterial von ca. 50g enthält 74% Δ-9-Tetra-hydrocannabinol (Delta-9-THC), 25% Nebenprodukte sowie <1% Cannabidiol. Nach säulenchromatographischer Aufreinigung erhält man 30g reines Delta-9-THC, was einer Ausbeute von 60% d.Th. entspricht.

Die Schritte der Synthese von Delta-9-THC via 2-Hydroxyethylcannabidiolat (III) sind nachfolgend schematisch dargestellt:

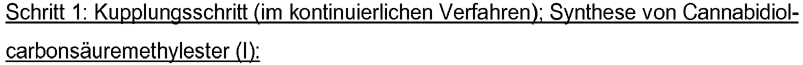

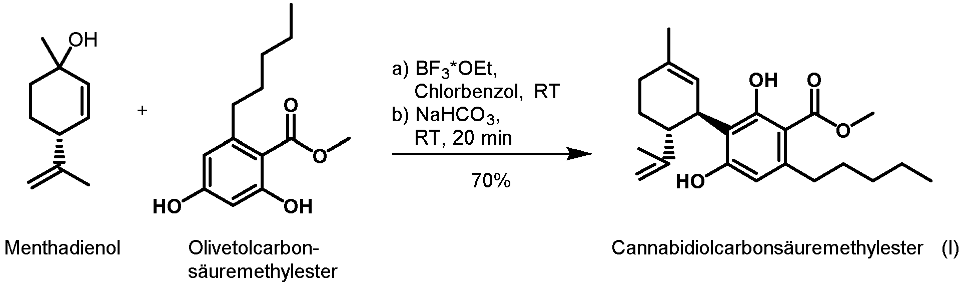

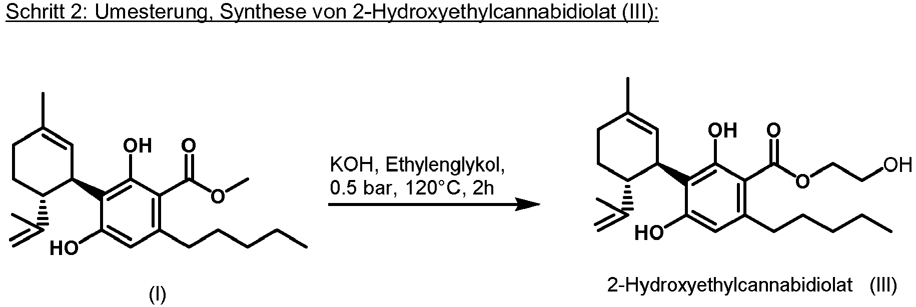

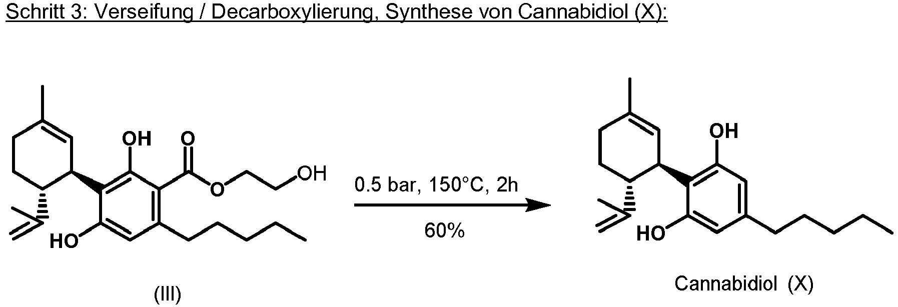

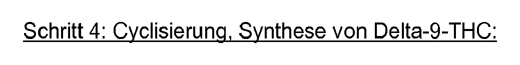

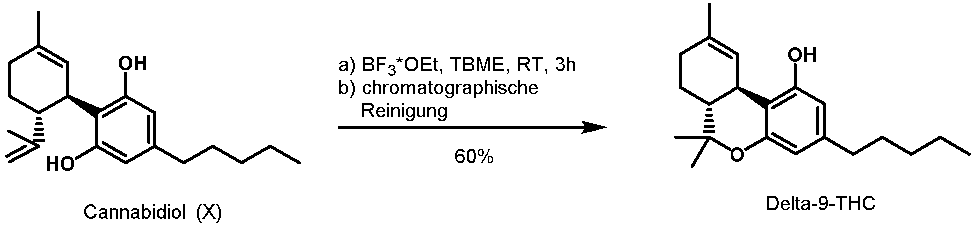

### C. Anwendungsbeispiele:

Anhand der nachfolgenden Beispiele von bevorzugten erfindungsgemäßen pharmazeutischen Formulierungen wird die erfindungsgemäße Anwendung von Verbindungen der Formel (A) näher erläutert. Bevorzugt ist insoweit der Einsatz von Verbindung (V).

Anwendungsbeispiel 1 - Kapseln nach dem *"*Neuen Rezeptur Formularium", 18. Ergänzung, 2001.

| Ansatz für 1 Kapsel | | | |
|---|---|---|---|
| | 2,5 mg | 5 mg | 10 mg |
| Verbindung der Formel (A) | 0,0025 g | 0,005 g | 0,010 g |
| Hartfett (Steigschmelzp.: 37-40 °C; OH-Zahl: 7-17; VS-Zahl: 245-260) | zu 0,430 g | zu 0,430 g | zu 0,430 g |
| Hartgelatine-Steckkapselhülle, Größe 1 | 1 Stück | 1 Stück | 1 Stück |

| Ansatz für 30 Kapseln einschließlich 10 % Überschuss der Schmelze | | | |
|---|---|---|---|
| | 2,5 mg | 5 mg | 10 mg |
| Verbindung der Formel (A) | 0,083 g | 0,165 g | 0,33 g |
| Hartfett (Steigschmelzp.: 37-40 °C; OH-Zahl: 7-17; VS-Zahl: 245-260) | zu 14,2 g | zu 14,2 g | zu 14,2 g |
| Hartgelatine-Steckkapselhüllen, Größe 1 | 30 Stück | 30 Stück | 30 Stück |

| Ansatz für 60 Kapseln einschließlich 5 % Überschuss der Schmelze | | | |
|---|---|---|---|
| | 2,5 mg | 5 mg | 10 mg |
| Verbindung der Formel (A) | 0,158 g | 0,315 g | 0,63 g |
| Hartfett (Steigschmelzp.: 37-40 °C; OH-Zahl: 7-17; VS-Zahl: 245-260) | zu 27,1 g | zu 27,1 g | zu 27,1 g |
| Hartgelatine-Steckkapselhüllen, Größe 1 | 60 Stück | 60 Stück | 60 Stück |

1. In einer waagerecht justierten Kapselfüllmaschine werden die eingesetzten Hartgelatine-Steckkapselhüllen geöffnet, die fixierten Kapselunterteile freigelegt und zur Befüllung bereitgestellt.
2. In einem Becherglas wird etwas mehr Hartfett als für den Ansatz benötigt auf dem Wasserbad geschmolzen. Inprozessprüfung: Die Hartfettschmelze muss bei visueller Prüfung klar sein. Sie darf schwach gelb gefärbt sein.
3. In einem zweiten Becherglas wird zu der Verbindung der Formel (A) bis zur angegebenen Ansatzmenge geschmolzenes Hartfett hinzugegeben. Die Substanz wird unter Rühren mit einem Glassstab gelöst. Inprozessprüfung: Die Fettschmelze muss bei visueller Prüfung klar sein. Sie darf schwach gelb gefärbt sein.
4. Die Fettschmelze wird bis zur Befüllung der letzten Kapseln auf dem noch warmen, aber nicht mehr siedenden Wasserbad stehen gelassen, oder sie wird aus dem Wasserbad genommen und bei Bedarf wieder erwärmt. Inprozessprüfung (gelegentlich zu wiederholen): Die Temperatur der Schmelze muss zwischen 35 und 45 °C liegen.
5. Etwa 1 ml Fettschmelze wird über eine möglichst weitlumige Kanüle in eine 1-ml-Einmalspritze aufgezogen (siehe unter "Pharmazeutische Erläuterungen - Herstellungstechnik und Abfüllung"). Unverzüglich werden zwei Kapselunterteile befüllt.

Inprozessprüfung: Der obere Rand des Kapselunterteils muss von innen vollständig mit Fettschmelze benetzt sein. Die Flüssigkeitsoberfläche muss plan oder schwach nach innen gewölbt (konkav) sein.
6. Die Spritze wird erneut befüllt, und die Füllung weiterer Kapseln wird so lange fortgesetzt, bis alle Kapselunterteile befüllt sind. Der beim Erkalten der Schmelze in den Kapseln entstehende Leerraum darf nicht weiter aufgefüllt werden. Inprozessprüfung: Im Becherglas darf nur ein kleiner Rest von etwa 1 ml Fettschmelze übrig bleiben.
7. Nach Erstarren der Fettschmelze in den Kapselunterteilen werden die Kapseln fest verschlossen.

Inprozessprüfung: Die Oberfläche der Fettschmelze muss in allen Kapselunterteilen gleichartig opak aussehen.

Endproduktprüfungen: Die verschlossenen Kapseln müssen gleichmäßig aussehen. Nur bei Bedarf: Die Einzelmassen aller Kapseln müssen zwischen 460 und 540 mg liegen.

Anwendungsbeispiel 2 - Ölige Tropfen nach dem "Neuen Rezeptur Formularium", 19. Ergänzung, 2002.

| Bestandteile | | |
|---|---|---|
| | 20 g 100 Masseteile | |
| Verbindung der Formel (A) (siehe Bezugsquellennachweis für Rezepturbestandteile, Abschnitt III.2.) | 0,5 g | 2,5 Teile |
| Mittelkettige Triglyceride | zu 20,0 g | zu 100,0 Teilen |

1. Die Verbindung der Formel (A) wird im Vorratsbehältnis durch gelinde Erwärmung verflüssigt.
2. Die Verbindung der Formel (A) wird in ein Becherglas gewogen und unter Erwärmung und Rühren in den mittelkettigen Triglyceriden gelöst.

Endproduktprüfung: Die Lösung muss bei visueller Prüfung klar sein. Sie darf schwach gelb gefärbt sein.

## Patentansprüche

1. Mischung umfassend eine oder mehrere Verbindungen der Formel (A) und/oder ein oder mehrere von deren Salzen, 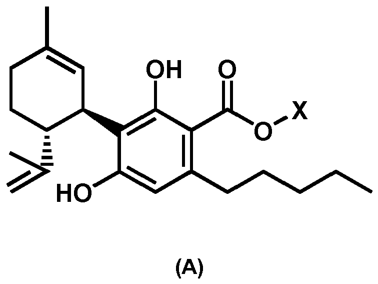 wobei X ein aliphatischer Rest mit einer, zwei, drei oder mehr als drei Hydroxygruppen ist, wobei die Gesamtzahl der C-Atome in dem aliphatischen Rest X nicht größer ist als 15, und
wobei der aliphatische Rest
- gesättigt oder ungesättigt ist
und
- verzweigt oder unverzweigt ist,
wobei in der Mischung
das molare Verhältnis der Gesamtmenge an Verbindungen der Formel (A) und deren Salzen zur Menge an Cannabidiol größer ist als 1 : 1, und gleichzeitig
das molare Verhältnis der Gesamtmenge an Verbindungen der Formel (A) und deren Salzen zur Menge an der Verbindung der Formel (I) 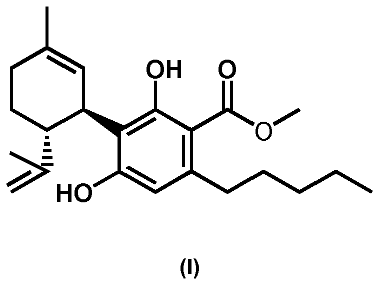 größer ist als 1 : 1,
wobei die Verbindung der Formel (A) ausgewählt ist aus der Gruppe bestehend aus:

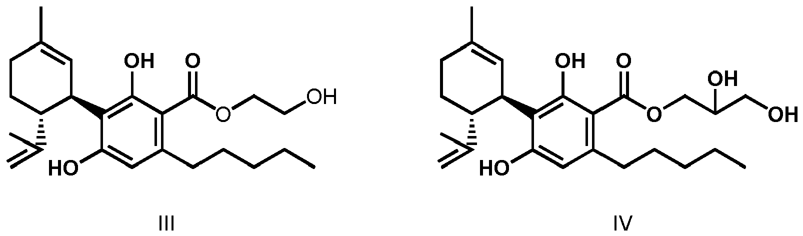

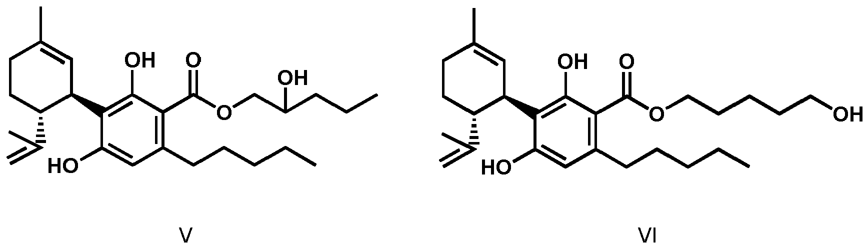

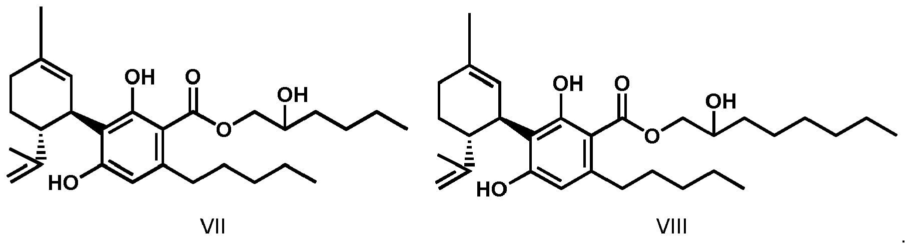

2. Verbindung der Formel (A) wie in Anspruch 1 definiert oder Salz einer Verbindung der Formel (A) wie in Anspruch 1 definiert oder Mischung nach Anspruch 1 zur Anwendung als Arzneimittel.

3. Verbindung der Formel (A) wie in Anspruch 1 definiert oder Salz einer Verbindung der Formel (A) wie in Anspruch 1 definiert oder Mischung nach Anspruch 1 zur Anwendung in einem Verfahren zur therapeutischen Behandlung des menschlichen oder tierischen Körpers

4. Verbindung der Formel (A) wie in Anspruch 1 definiert oder Salz einer Verbindung der Formel (A) wie in Anspruch 1 definiert oder Mischung nach Anspruch 1, zur spezifischen Anwendung in einem Verfahren zur therapeutischen Behandlung des menschlichen oder tierischen Körpers, zum Erreichen einer Wirkung ausgewählt aus der Gruppe bestehend aus
- appetitanregende Wirkung,
- antiemetische Wirkung zur Hemmung von Übelkeit und Erbrechen,
- Reduktion muskulärer Krämpfe und Spastiken,
- Linderung von Schmerzsymptomen
- Linderung von Migränesymptomen,
- Senkung des Augeninnendrucks beim Glaukom,
- Stimmungsaufhellung
- Immunstimulation
und/oder
- antiepileptische Wirkung.

5. Pharmazeutische Formulierung, umfassend eine oder mehrere Verbindungen der Formel (A) wie in Anspruch 1 definiert oder umfassend ein oder mehrere von deren Salzen wie in Anspruch 1 definiert oder umfassend eine Mischung gemäß Anspruch 1.

6. Verfahren zur Herstellung einer Mischung gemäß Anspruch 1, mit folgendem Schritt:
Umsetzen eines Cannabidiolcarbonsäureesters der Formel (IX) 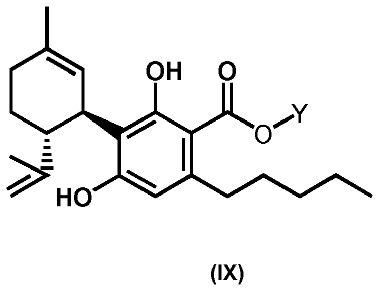 wobei Y ein organischer Rest ist,
mit einem Alkohol der Formel HO-X,
wobei
X ein aliphatischer Rest mit einer, zwei, drei oder mehr als drei Hydroxygruppen ist, wobei die Gesamtzahl der C-Atome in dem aliphatischen Rest X nicht größer ist als 15, und wobei der aliphatische Rest
- gesättigt oder ungesättigt ist
und
- verzweigt oder unverzweigt ist,
wobei Y verschieden von X ist und so gewählt ist, dass bei der Umsetzung entstehender Alkohol der Formel HO-Y bei 1013 hPa bei niedrigerer Temperatur siedet als der eingesetzte Alkohol der Formel HO-X.

7. Verfahren nach Anspruch 6, mit folgendem Schritt zur Herstellung des Cannabidiolcarbonsäureesters der Formel (IX):
Umsetzen von Menthadienol mit einem Olivetolcarbonsäureester zum entsprechenden Cannabidiolcarbonsäureester der Formel (IX), vorzugsweise in einem kontinuierlichen Verfahren.

8. Verfahren zur Herstellung von Delta-9-Tetrahydrocannabinol, umfassend die Herstellung einer Mischung gemäß Anspruch 1 mittels eines Verfahrens nach einem der Ansprüche 6 bis 7.

9. Verfahren nach Anspruch 8, wobei die hergestellte Mischung gemäß Anspruch 1 so behandelt wird, dass die in der Mischung enthaltene Verbindung der Formel (A) decarboxylierend verseift und die Verbindung (X) gebildet wird.

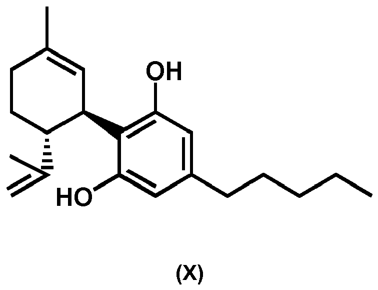

10. Verfahren nach einem der Ansprüche 8 oder 9, wobei die nach decarboxylierender Verseifung vorliegende Verbindung (X) zu Delta-9-Tetrahydrocannabinol cyclisiert wird, vorzugsweise in Abwesenheit halogenhaltiger Lösungsmittel.

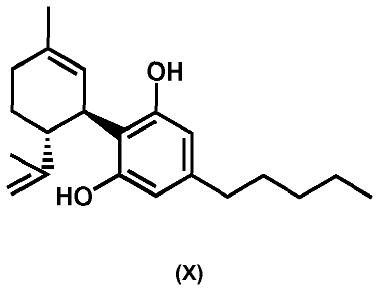

## Claims

1. A composition comprising one or multiple compound(s) of formula (A) and/or one or multiple salt(s) thereof, 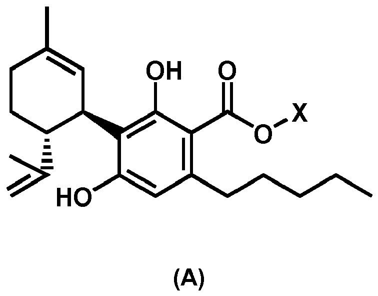 wherein X is an aliphatic residue with one, two, three or more than three hydroxyl groups, wherein the total number of C-atoms in the aliphatic residue X is not greater than 15, and
wherein the aliphatic residue
- is saturated or unsaturated,
and
- is branched or unbranched,
wherein in the composition
the molar ratio of the total amount of compounds of formula (A) and salts thereof to the amount of cannabidiol is greater than 1 : 1, and simultaneously
the molar ratio of the total amount of compounds of formula (A) and salts thereof to the amount of compounds of formula (I) 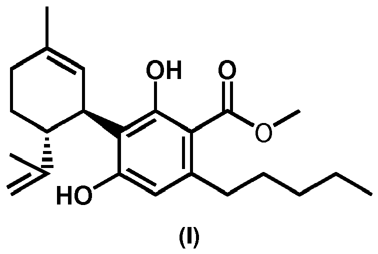 is greater than 1 : 1,
wherein the compound of formula (A) is selected from the group consisting of:

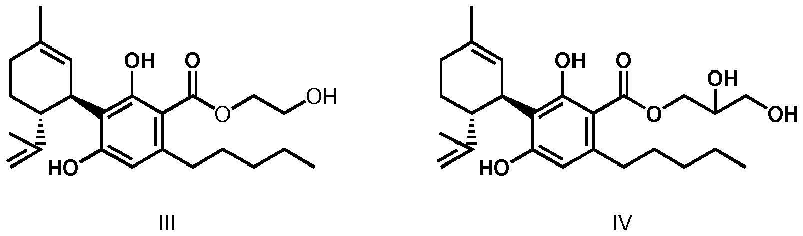

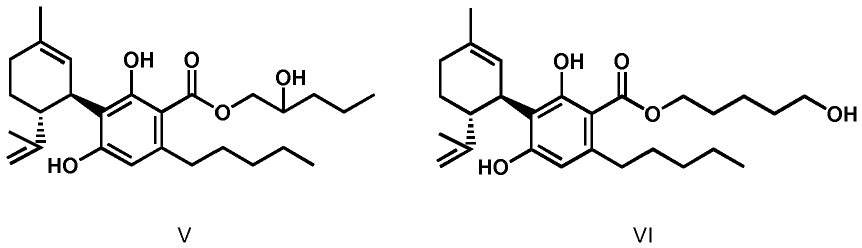

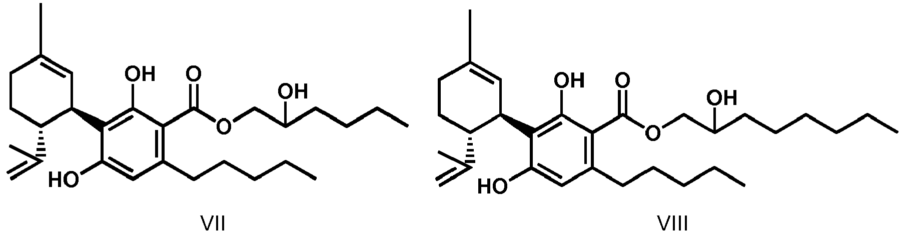

2. The compound of formula (A) as defined in claim 1 or salt of a compound of formula (A) as defined in claim 1 or composition according to claim 1 for use as medicine.

3. The compound of formula (A) as defined in claim 1 or salt of a compound of formula (A) as defined in claim 1 or composition according to claim 1 for use in a method for the therapeutic treatment of the human or animal body.

4. The compound of formula (A) as defined in claim 1 or salt of a compound of formula (A) as defined in claim 1 or composition according to claim 1 for specific use in a method for the therapeutic treatment of the human or animal body to achieve an effect selected from the group consisting of
- appetite-stimulating effect,
- anti-emetic effect to inhibit nausea and vomiting,
- reduction of muscular cramps and spasticity,
- alleviation of pain symptoms,
- alleviation of migraine symptoms,
- reduction of intraocular pressure related to glaucoma,
- mood enhancement,
- immunostimulation
and/or
- antiepileptic effect.

5. Pharmaceutical formulation comprising one or multiple compound(s) of formula (A) as defined in claim 1 or comprising one or multiple salt(s) thereof as defined in claim 1 or comprising a composition according to claim 1.

6. The method for the manufacture of a composition according to claim 1 including the following step:
Conversion of a cannabidiolic acid ester of formula (IX) 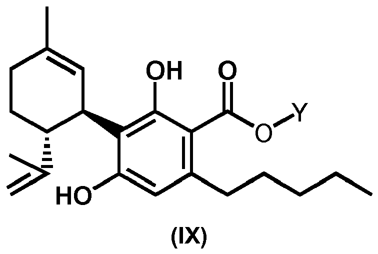 wherein Y is an organic residue,
with an alcohol of the formula HO-X,
wherein
X is an aliphatic residue with one, two, three or more than three hydroxyl groups, wherein the total number of C-atoms in the aliphatic residue X is not greater than 15, and
wherein the aliphatic residue is
- saturated or unsaturated
and
- branched or unbranched,
wherein Y is different from X and selected such that the alcohol of formula HO-Y, which is generated during the conversion, boils at a lower temperature at 1013 hPa than the used alcohol of formula HO-X.

7. The method according to claim 6 including the following step for manufacture of the cannabidiolic acid ester of formula (IX):
Conversion of menthadienol with an olivetolic acid ester to the corresponding cannabidiolic acid ester of formula (IX), preferably in a continuous process.

8. The method for the manufacture of delta-9-tetrahydrocannabinol comprising the manufacture of a composition according to claim 1 by means of a method according to any one of the claims 6 to 7.

9. A method according to claim 8, wherein the manufactured composition according to claim 1 is treated in such a way that the compound of formula (A) that is contained in the composition is decarboxylated and saponified to generate compound (X).

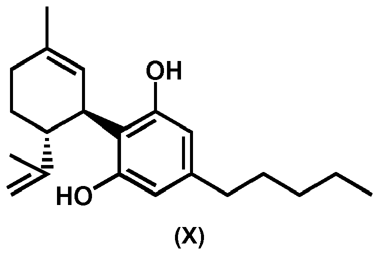

10. A method according to claim 8 or 9, wherein compound (X), which is present after the decarboxylating saponification, is cyclised to delta-9-tetrahydrocannabinol, preferably in the absence of halogenated solvents.

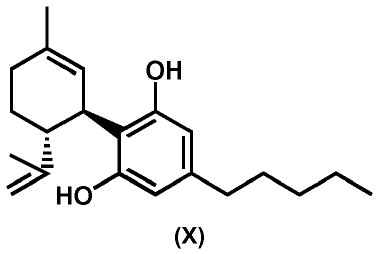

## Revendications

1. Mélange comprenant un ou plusieurs composés de formule (A) et/ou un ou plusieurs de ses/leurs sels, 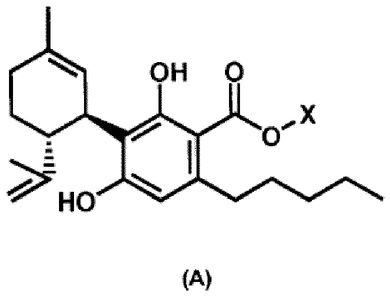 où X est un radical aliphatique comportant un, deux, trois ou plus de trois groupes hydroxy, le nombre total des atomes de carbone dans le radical aliphatique X n'étant pas supérieur à 15, et
le radical aliphatique
- étant saturé ou insaturé
et
- étant ramifié ou non ramifié,
dans le mélange
le rapport molaire de la quantité totale des composés de formule (A) et de leurs sels à la quantité de cannabidiol étant supérieur à 1 : 1, et en même temps
le rapport molaire de la quantité totale des composés de formule (A) et de leurs sels à la quantité du composé de formule (I) 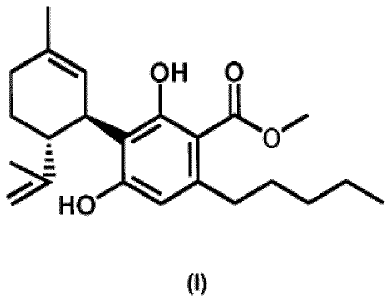 étant supérieur à 1 : 1,
le composé de formule (A) étant choisi dans le groupe constitué par :

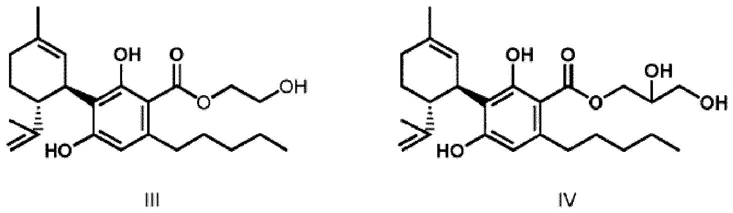

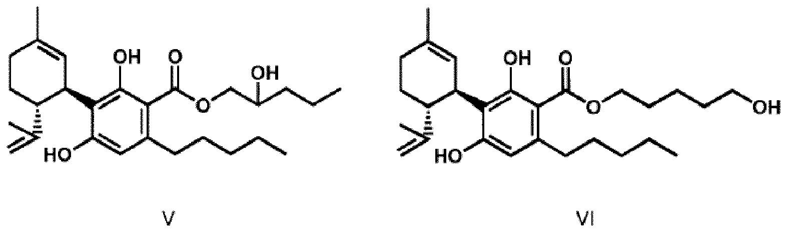

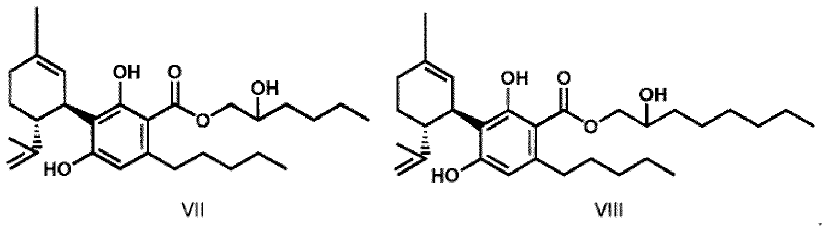

2. Composé de formule (A) tel que défini dans la revendication 1 ou sel d'un composé de formule (A) tel que défini dans la revendication 1 ou mélange selon la revendication 1, pour utilisation en tant que médicament.

3. Composé de formule (A) tel que défini dans la revendication 1 ou sel d'un composé de formule (A) tel que défini dans la revendication 1 ou mélange selon la revendication 1, pour utilisation dans un procédé pour le traitement thérapeutique du corps humain ou animal.

4. Composé de formule (A) tel que défini dans la revendication 1 ou sel d'un composé de formule (A) tel que défini dans la revendication 1 ou mélange selon la revendication 1, pour utilisation spécifique dans un procédé pour le traitement thérapeutique du corps humain ou animal, pour l'obtention d'un effet choisi dans le groupe constitué par
- un effet stimulant l'appétit,
- un effet antiémétique pour l'inhibition des nausées et des vomissements,
- la réduction des spasmes et crampes musculaires,
- l'atténuation de symptômes douloureux,
- l'atténuation de symptômes migraineux,
- l'abaissement de la pression intraoculaire dans le glaucome,
- l'amélioration de l'humeur,
- la stimulation des défenses immunitaires
et/ou
- un effet antiépileptique.

5. Composition pharmaceutique, comprenant un ou plusieurs composés de formule (A) tel que défini dans la revendication 1 ou comprenant un ou plusieurs de leurs sels tels que définis dans la revendication 1 ou comprenant un mélange selon la revendication 1.

6. Procédé pour la préparation d'un mélange selon la revendication 1, comprenant l'étape suivante :
mise en réaction d'un ester d'acide cannabidiolcarboxylique de formule (IX) 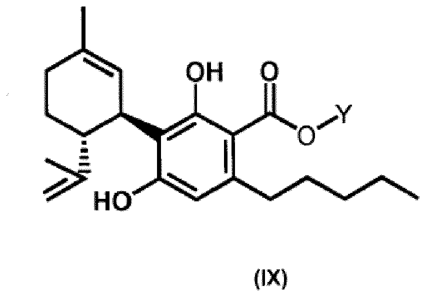 dans laquelle Y est un radical organique,
avec un alcool de formule HO-X,
dans laquelle
X est un radical aliphatique comportant un, deux, trois ou plus de trois groupes hydroxy, le nombre total des atomes de carbone dans le radical aliphatique X n'étant pas supérieur à 15, et
le radical aliphatique
- étant saturé ou insaturé
et
- étant ramifié ou non ramifié,
Y étant différent de X et étant choisi de manière que l'alcool de formule HO-Y formé dans la réaction ait sous 1 013 hPa une plus basse température d'ébullition que l'alcool de formule HO-X utilisé.

7. Procédé selon la revendication 6, comportant l'étape suivante dans la préparation de l'ester d'acide cannabidiolcarboxylique de formule (IX) :
mise en réaction de menthadiénol avec un ester d'acide olivétolcarboxylique, conduisant à l'ester d'acide cannabidiolcarboxylique de formule (IX) correspondant, de préférence dans un procédé continu.

8. Procédé pour la préparation de delta-9-tétrahydrocannabinol, comprenant la préparation d'un mélange selon la revendication 1 au moyen d'un procédé selon l'une quelconque des revendications 6 et 7.

9. Procédé selon la revendication 8, dans lequel on traite le mélange selon la revendication 1, préparé, de manière que le composé de formule (A) contenu dans le mélange soit saponifié par saponification décarboxylante et le composé (X) soit formé.

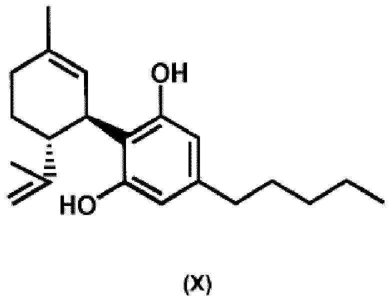

10. Procédé selon l'une quelconque des revendications 8 et 9, dans lequel le composé (X) présent après saponification décarboxylante est cyclisé en delta-9-tétrahydrocannabinol, de préférence en absence de solvants halogénés.
